(19)

Europäisches
Patentamt
European
Patent Office
Office européen
des brevets

(11)  **EP 2 664 923 B1**

(12)  # EUROPEAN PATENT SPECIFICATION

(45) Date of publication and mention
of the grant of the patent:
**18.02.2015  Bulletin 2015/08**

(51) Int Cl.:
**G01N 33/68** (2006.01)    **A61K 39/00** (2006.01)
**C12N 15/00** (2006.01)

(21) Application number: **13168110.8**

(22) Date of filing: **16.05.2013**

(54) **Biomarkers for central nervous system diseases**

Biomarker für Erkrankungen des Zentralnervensystems

Biomarqueurs pour des maladies du système nerveux central

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.05.2012  IT MI20120865**

(43) Date of publication of application:
**20.11.2013  Bulletin 2013/47**

(73) Proprietors:
• **Fondazione Italiana Sclerosi Multipla - FISM
Onlus
16149 Genova (IT)**
• **Bertolotto, Antonio
10126 Torino (IT)**

(72) Inventors:
• **Bertolotto, Antonio
I-10126 Torino (IT)**
• **Perga, Simona
I-10070 Cafasse (Torino) (IT)**
• **Giuliano Albo, Alessandra
I-10132 Torino (IT)**

• **Corpillo, Davide
I-10080 Pecco (Torino) (IT)**

(74) Representative: **Long, Giorgio et al
Jacobacci & Partners S.p.A.
Via Senato 8
20121 Milano (IT)**

(56) References cited:
**WO-A2-02/059604**

• **OTTERVALD J ET AL: "Multiple sclerosis:
Identification and clinical evaluation of novel CSF
biomarkers", JOURNAL OF PROTEOMICS,
ELSEVIER, AMSTERDAM, NL, vol. 73, no. 6, 18
April 2010 (2010-04-18) , pages 1117-1132,
XP027098857, ISSN: 1874-3919 [retrieved on
2010-04-18]**
• **GIULIO DISANTO ET AL: "The emerging role of
vitamin D binding protein in multiple sclerosis",
JOURNAL OF NEUROLOGY, STEINKOPFF-
VERLAG, DA, vol. 258, no. 3, 2 November 2010
(2010-11-02), pages 353-358, XP019887301, ISSN:
1432-1459, DOI: 10.1007/S00415-010-5797-8**

EP 2 664 923 B1

SEQUENCE LISTING

<110> FISM Bertolotto, Antonio

<120> Biomarcatori per patologie del sistema nervoso centrale

<130> I0149096

<160> 4

<170> PatentIn version 3.5

<210> 1
<211> 782
<212> PRT
<213> Homo sapiens

<400> 1

```
Met Ala Pro His Arg Pro Ala Pro Ala Leu Leu Cys Ala Leu Ser Leu
1               5                   10                  15

Ala Leu Cys Ala Leu Ser Leu Pro Val Arg Ala Ala Thr Ala Ser Arg
            20                  25                  30

Gly Ala Ser Gln Ala Gly Ala Pro Gln Gly Arg Val Pro Glu Ala Arg
            35                  40                  45

Pro Asn Ser Met Val Val Glu His Pro Glu Phe Leu Lys Ala Gly Lys
        50                  55                  60

Glu Pro Gly Leu Gln Ile Trp Arg Val Glu Lys Phe Asp Leu Val Pro
65                  70                  75                  80

Val Pro Thr Asn Leu Tyr Gly Asp Phe Phe Thr Gly Asp Ala Tyr Val
            85                  90                  95

Ile Leu Lys Thr Val Gln Leu Arg Asn Gly Asn Leu Gln Tyr Asp Leu
            100                 105                 110

His Tyr Trp Leu Gly Asn Glu Cys Ser Gln Asp Glu Ser Gly Ala Ala
        115                 120                 125

Ala Ile Phe Thr Val Gln Leu Asp Asp Tyr Leu Asn Gly Arg Ala Val
    130                 135                 140

Gln His Arg Glu Val Gln Gly Phe Glu Ser Ala Thr Phe Leu Gly Tyr
145                 150                 155                 160

Phe Lys Ser Gly Leu Lys Tyr Lys Lys Gly Gly Val Ala Ser Gly Phe
            165                 170                 175
```

Lys His Val Val Pro Asn Glu Val Val Val Gln Arg Leu Phe Gln Val
            180                 185                 190

Lys Gly Arg Arg Val Val Arg Ala Thr Glu Val Pro Val Ser Trp Glu
            195                 200                 205

Ser Phe Asn Asn Gly Asp Cys Phe Ile Leu Asp Leu Gly Asn Asn Ile
    210                 215                 220

His Gln Trp Cys Gly Ser Asn Ser Asn Arg Tyr Glu Arg Leu Lys Ala
225                 230                 235                 240

Thr Gln Val Ser Lys Gly Ile Arg Asp Asn Glu Arg Ser Gly Arg Ala
                245                 250                 255

Arg Val His Val Ser Glu Glu Gly Thr Glu Pro Glu Ala Met Leu Gln
            260                 265                 270

Val Leu Gly Pro Lys Pro Ala Leu Pro Ala Gly Thr Glu Asp Thr Ala
            275                 280                 285

Lys Glu Asp Ala Ala Asn Arg Lys Leu Ala Lys Leu Tyr Lys Val Ser
    290                 295                 300

Asn Gly Ala Gly Thr Met Ser Val Ser Leu Val Ala Asp Glu Asn Pro
305                 310                 315                 320

Phe Ala Gln Gly Ala Leu Lys Ser Glu Asp Cys Phe Ile Leu Asp His
            325                 330                 335

Gly Lys Asp Gly Lys Ile Phe Val Trp Lys Gly Lys Gln Ala Asn Thr
            340                 345                 350

Glu Glu Arg Lys Ala Ala Leu Lys Thr Ala Ser Asp Phe Ile Thr Lys
            355                 360                 365

Met Asp Tyr Pro Lys Gln Thr Gln Val Ser Val Leu Pro Glu Gly Gly
    370                 375                 380

Glu Thr Pro Leu Phe Lys Gln Phe Phe Lys Asn Trp Arg Asp Pro Asp
385                 390                 395                 400

Gln Thr Asp Gly Leu Gly Leu Ser Tyr Leu Ser Ser His Ile Ala Asn
            405                 410                 415

Val Glu Arg Val Pro Phe Asp Ala Ala Thr Leu His Thr Ser Thr Ala
            420                 425                 430

16

Met Ala Ala Gln His Gly Met Asp Asp Asp Gly Thr Gly Gln Lys Gln
    435            440            445

Ile Trp Arg Ile Glu Gly Ser Asn Lys Val Pro Val Asp Pro Ala Thr
    450            455            460

Tyr Gly Gln Phe Tyr Gly Gly Asp Ser Tyr Ile Ile Leu Tyr Asn Tyr
465              470          475          480

Arg His Gly Gly Arg Gln Gly Gln Ile Ile Tyr Asn Trp Gln Gly Ala
          485          490          495

Gln Ser Thr Gln Asp Glu Val Ala Ala Ser Ala Ile Leu Thr Ala Gln
        500          505          510

Leu Asp Glu Glu Leu Gly Gly Thr Pro Val Gln Ser Arg Val Val Gln
        515          520          525

Gly Lys Glu Pro Ala His Leu Met Ser Leu Phe Gly Gly Lys Pro Met
    530          535          540

Ile Ile Tyr Lys Gly Gly Thr Ser Arg Glu Gly Gly Gln Thr Ala Pro
545            550          555          560

Ala Ser Thr Arg Leu Phe Gln Val Arg Ala Asn Ser Ala Gly Ala Thr
        565          570          575

Arg Ala Val Glu Val Leu Pro Lys Ala Gly Ala Leu Asn Ser Asn Asp
        580          585          590

Ala Phe Val Leu Lys Thr Pro Ser Ala Ala Tyr Leu Trp Val Gly Thr
        595          600          605

Gly Ala Ser Glu Ala Glu Lys Thr Gly Ala Gln Glu Leu Leu Arg Val
    610          615          620

Leu Arg Ala Gln Pro Val Gln Val Ala Glu Gly Ser Glu Pro Asp Gly
625            630          635          640

Phe Trp Glu Ala Leu Gly Gly Lys Ala Ala Tyr Arg Thr Ser Pro Arg
        645          650          655

Leu Lys Asp Lys Lys Met Asp Ala His Pro Pro Arg Leu Phe Ala Cys
        660          665          670

Ser Asn Lys Ile Gly Arg Phe Val Ile Glu Glu Val Pro Gly Glu Leu

675 680 685

Met Gln Glu Asp Leu Ala Thr Asp Asp Val Met Leu Leu Asp Thr Trp
690 695 700

Asp Gln Val Phe Val Trp Val Gly Lys Asp Ser Gln Glu Glu Glu Lys
705 710 715 720

Thr Glu Ala Leu Thr Ser Ala Lys Arg Tyr Ile Glu Thr Asp Pro Ala
725 730 735

Asn Arg Asp Arg Arg Thr Pro Ile Thr Val Val Lys Gln Gly Phe Glu
740 745 750

Pro Pro Ser Phe Val Gly Trp Phe Leu Gly Trp Asp Asp Asp Tyr Trp
755 760 765

Ser Val Asp Pro Leu Asp Arg Ala Met Ala Glu Leu Ala Ala
770 775 780

<210> 2
<211> 782
<212> PRT
<213> Homo sapiens

<400> 2

Met Ala Pro His Arg Pro Ala Pro Ala Leu Leu Cys Ala Leu Ser Leu
1 5 10 15

Ala Leu Cys Ala Leu Ser Leu Pro Val Arg Ala Ala Thr Ala Ser Arg
20 25 30

Gly Ala Ser Gln Ala Gly Ala Pro Gln Gly Arg Val Pro Glu Ala Arg
35 40 45

Pro Asn Ser Met Val Val Glu His Pro Glu Phe Leu Lys Ala Gly Lys
50 55 60

Glu Pro Gly Leu Gln Ile Trp Arg Val Glu Lys Phe Asp Leu Val Pro
65 70 75 80

Val Pro Thr Asn Leu Tyr Gly Asp Phe Phe Thr Gly Asp Ala Tyr Val
85 90 95

Ile Leu Lys Thr Val Gln Leu Arg Asn Gly Asn Leu Gln Tyr Asp Leu
100 105 110

His Tyr Trp Leu Gly Asn Glu Cys Ser Gln Asp Glu Ser Gly Ala Ala

18

115                   120                   125

Ala Ile Phe Thr Val Gln Leu Asp Asp Tyr Leu Asn Gly Arg Ala Val
    130              135            140

Gln His Arg Glu Val Gln Gly Phe Glu Ser Ala Thr Phe Leu Gly Tyr
145              150            155            160

Phe Lys Ser Gly Leu Lys Tyr Lys Lys Gly Gly Val Ala Ser Gly Phe
           165           170            175

Lys His Val Val Pro Asn Glu Val Val Val Gln Arg Leu Phe Gln Val
         180           185           190

Lys Gly Arg Arg Val Val Arg Ala Thr Glu Val Pro Val Ser Trp Glu
        195           200          205

Ser Phe Asn Asn Gly Asp Cys Phe Ile Leu Asp Leu Gly Asn Asn Ile
    210              215            220

His Gln Trp Cys Gly Ser Asn Ser Asn Arg Tyr Glu Arg Leu Lys Ala
225              230          235            240

Thr Gln Val Ser Lys Gly Ile Arg Asp Asn Glu Arg Ser Gly Arg Ala
         245           250          255

Arg Val His Val Ser Glu Glu Gly Thr Glu Pro Glu Ala Met Leu Gln
        260          265          270

Val Leu Gly Pro Lys Pro Ala Leu Pro Ala Gly Thr Glu Asp Thr Ala
        275          280          285

Lys Glu Asp Ala Ala Asn Arg Lys Leu Ala Lys Leu Tyr Lys Val Ser
    290              295          300

Asn Gly Ala Gly Thr Met Ser Val Ser Leu Val Ala Asp Glu Asn Pro
305              310          315          320

Phe Ala Gln Gly Ala Leu Lys Ser Glu Asp Cys Phe Ile Leu Asp His
         325           330          335

Gly Lys Asp Gly Lys Ile Phe Val Trp Lys Gly Lys Gln Ala Asn Thr
        340          345          350

Glu Glu Arg Lys Ala Ala Leu Lys Thr Ala Ser Asp Phe Ile Thr Lys
       355           360          365

```
Met Asp Tyr Pro Lys Gln Thr Gln Val Ser Val Leu Pro Glu Gly Gly
    370         375             380

Glu Thr Pro Leu Phe Lys Gln Phe Phe Lys Asn Trp Arg Asp Pro Asp
385             390             395             400

Gln Thr Asp Gly Leu Gly Leu Ser Tyr Leu Ser Ser His Ile Ala Asn
            405             410             415

Val Glu Arg Val Pro Phe Asp Ala Ala Thr Leu His Thr Ser Thr Ala
            420             425             430

Met Ala Ala Gln His Gly Met Asp Asp Asp Gly Thr Gly Gln Lys Gln
        435             440             445

Ile Trp Arg Ile Glu Gly Ser Asn Lys Val Pro Val Asp Pro Ala Thr
    450             455             460

Tyr Gly Gln Phe Tyr Gly Gly Asp Ser Tyr Ile Ile Leu Tyr Asn Tyr
465             470             475             480

Arg His Gly Gly Arg Gln Gly Gln Ile Ile Tyr Asn Trp Gln Gly Ala
            485             490             495

Gln Ser Thr Gln Asp Glu Val Ala Ala Ser Ala Ile Leu Thr Ala Gln
        500             505             510

Leu Asp Glu Glu Leu Gly Gly Thr Pro Val Gln Ser Arg Val Val Gln
        515             520             525

Gly Lys Glu Pro Ala His Leu Met Ser Leu Phe Gly Gly Lys Pro Met
    530             535             540

Ile Ile Tyr Lys Gly Gly Thr Ser Arg Glu Gly Gly Gln Thr Ala Pro
545             550             555             560

Ala Ser Thr Arg Leu Phe Gln Val Arg Ala Asn Ser Ala Gly Ala Thr
            565             570             575

Arg Ala Val Glu Val Leu Pro Lys Ala Gly Ala Leu Asn Ser Asn Asp
            580             585             590

Ala Phe Val Leu Lys Thr Pro Ser Ala Ala Tyr Leu Trp Val Gly Thr
        595             600             605

Gly Ala Ser Glu Ala Glu Lys Thr Gly Ala Gln Glu Leu Leu Arg Val
    610             615             620
```

```
Leu Arg Ala Gln Pro Val Gln Val Ala Glu Gly Ser Glu Pro Asp Gly
625             630             635             640

Phe Trp Glu Ala Leu Gly Gly Lys Ala Ala Tyr Arg Thr Ser Pro Arg
                645             650             655

Leu Lys Asp Lys Lys Met Asp Ala His Pro Pro Arg Leu Phe Ala Cys
            660             665             670

Ser Asn Lys Ile Gly Arg Phe Val Ile Glu Glu Val Pro Gly Glu Leu
        675             680             685

Met Gln Glu Asp Leu Ala Thr Asp Asp Val Met Leu Leu Asp Thr Trp
    690             695             700

Asp Gln Val Phe Val Trp Val Gly Lys Asp Ser Gln Glu Glu Glu Lys
705             710             715             720

Thr Glu Ala Leu Thr Ser Ala Lys Arg Tyr Ile Glu Thr Asp Pro Ala
            725             730             735

Asn Arg Asp Arg Arg Thr Pro Ile Thr Val Val Lys Gln Gly Phe Glu
            740             745             750

Pro Pro Ser Phe Val Gly Trp Phe Leu Gly Trp Asp Asp Asp Tyr Trp
        755             760             765

Ser Val Asp Pro Leu Asp Arg Ala Met Ala Glu Leu Ala Ala
    770             775             780
```

<210> 3
<211> 474
<212> PRT
<213> Homo sapiens

<400> 3

```
Met Lys Arg Val Leu Val Leu Leu Leu Ala Val Ala Phe Gly His Ala
1               5               10              15

Leu Glu Arg Gly Arg Asp Tyr Glu Lys Asn Lys Val Cys Lys Glu Phe
            20              25              30

Ser His Leu Gly Lys Glu Asp Phe Thr Ser Leu Ser Leu Val Leu Tyr
        35              40              45

Ser Arg Lys Phe Pro Ser Gly Thr Phe Glu Gln Val Ser Gln Leu Val
    50              55              60
```

```
Lys Glu Val Val Ser Leu Thr Glu Ala Cys Cys Ala Glu Gly Ala Asp
65              70              75                      80

Pro Asp Cys Tyr Asp Thr Arg Thr Ser Ala Leu Ser Ala Lys Ser Cys
                85              90                      95

Glu Ser Asn Ser Pro Phe Pro Val His Pro Gly Thr Ala Glu Cys Cys
            100             105             110

Thr Lys Glu Gly Leu Glu Arg Lys Leu Cys Met Ala Ala Leu Lys His
        115             120             125

Gln Pro Gln Glu Phe Pro Thr Tyr Val Glu Pro Thr Asn Asp Glu Ile
    130             135             140

Cys Glu Ala Phe Arg Lys Asp Pro Lys Glu Tyr Ala Asn Gln Phe Met
145             150             155             160

Trp Glu Tyr Ser Thr Asn Tyr Glu Gln Ala Pro Leu Ser Leu Leu Val
            165             170             175

Ser Tyr Thr Lys Ser Tyr Leu Ser Met Val Gly Ser Cys Cys Thr Ser
        180             185             190

Ala Ser Pro Thr Val Cys Phe Leu Lys Glu Arg Leu Gln Leu Lys His
        195             200             205

Leu Ser Leu Leu Thr Thr Leu Ser Asn Arg Val Cys Ser Gln Tyr Ala
    210             215             220

Ala Tyr Gly Glu Lys Lys Ser Arg Leu Ser Asn Leu Ile Lys Leu Ala
225             230             235             240

Gln Lys Val Pro Thr Ala Asp Leu Glu Asp Val Leu Pro Leu Ala Glu
            245             250             255

Asp Ile Thr Asn Ile Leu Ser Lys Cys Cys Glu Ser Ala Ser Glu Asp
            260             265             270

Cys Met Ala Lys Glu Leu Pro Glu His Thr Val Lys Leu Cys Asp Asn
        275             280             285

Leu Ser Thr Lys Asn Ser Lys Phe Glu Asp Cys Cys Gln Glu Lys Thr
    290             295             300

Ala Met Asp Val Phe Val Cys Thr Tyr Phe Met Pro Ala Ala Gln Leu
305             310             315             320
```

22

```
Pro Glu Leu Pro Asp Val Arg Leu Pro Thr Asn Lys Asp Val Cys Asp
                325             330             335

Pro Gly Asn Thr Lys Val Met Asp Lys Tyr Thr Phe Glu Leu Ser Arg
            340             345             350

Arg Thr His Leu Pro Glu Val Phe Leu Ser Lys Val Leu Glu Pro Thr
        355             360             365

Leu Lys Ser Leu Gly Glu Cys Cys Asp Val Glu Asp Ser Thr Thr Cys
    370             375             380

Phe Asn Ala Lys Gly Pro Leu Leu Lys Lys Glu Leu Ser Ser Phe Ile
385             390             395             400

Asp Lys Gly Gln Glu Leu Cys Ala Asp Tyr Ser Glu Asn Thr Phe Thr
            405             410             415

Glu Tyr Lys Lys Lys Leu Ala Glu Arg Leu Lys Ala Lys Leu Pro Glu
            420             425             430

Ala Thr Pro Thr Glu Leu Ala Lys Leu Val Asn Lys Arg Ser Asp Phe
            435             440             445

Ala Ser Asn Cys Cys Ser Ile Asn Ser Pro Pro Leu Tyr Cys Asp Ser
    450             455             460

Glu Ile Asp Ala Glu Leu Lys Asn Ile Leu
465             470
```

<210> 4
<211> 474
<212> PRT
<213> Homo sapiens

<400> 4

```
Met Lys Arg Val Leu Val Leu Leu Leu Ala Val Ala Phe Gly His Ala
1               5               10              15

Leu Glu Arg Gly Arg Asp Tyr Glu Lys Asn Lys Val Cys Lys Glu Phe
            20              25              30

Ser His Leu Gly Lys Glu Asp Phe Thr Ser Leu Ser Leu Val Leu Tyr
        35              40              45

Ser Arg Lys Phe Pro Ser Gly Thr Phe Glu Gln Val Ser Gln Leu Val
    50              55              60
```

```
Lys Glu Val Val Ser Leu Thr Glu Ala Cys Cys Ala Glu Gly Ala Asp
65            70            75               80


Pro Asp Cys Tyr Asp Thr Arg Thr Ser Ala Leu Ser Ala Lys Ser Cys
            85            90               95


Glu Ser Asn Ser Pro Phe Pro Val His Pro Gly Thr Ala Glu Cys Cys
            100           105              110


Thr Lys Glu Gly Leu Glu Arg Lys Leu Cys Met Ala Ala Leu Lys His
        115           120           125


Gln Pro Gln Glu Phe Pro Thr Tyr Val Glu Pro Thr Asn Asp Glu Ile
    130           135           140


Cys Glu Ala Phe Arg Lys Asp Pro Lys Glu Tyr Ala Asn Gln Phe Met
145           150           155              160


Trp Glu Tyr Ser Thr Asn Tyr Glu Gln Ala Pro Leu Ser Leu Leu Val
            165           170           175


Ser Tyr Thr Lys Ser Tyr Leu Ser Met Val Gly Ser Cys Cys Thr Ser
        180           185           190


Ala Ser Pro Thr Val Cys Phe Leu Lys Glu Arg Leu Gln Leu Lys His
        195           200           205


Leu Ser Leu Leu Thr Thr Leu Ser Asn Arg Val Cys Ser Gln Tyr Ala
    210           215           220


Ala Tyr Gly Glu Lys Lys Ser Arg Leu Ser Asn Leu Ile Lys Leu Ala
225           230           235              240


Gln Lys Val Pro Thr Ala Asp Leu Glu Asp Val Leu Pro Leu Ala Glu
            245           250           255


Asp Ile Thr Asn Ile Leu Ser Lys Cys Cys Glu Ser Ala Ser Glu Asp
            260           265           270


Cys Met Ala Lys Glu Leu Pro Glu His Thr Val Lys Leu Cys Asp Asn
        275           280           285


Leu Ser Thr Lys Asn Ser Lys Phe Glu Asp Cys Cys Gln Glu Lys Thr
    290           295           300


Ala Met Asp Val Phe Val Cys Thr Tyr Phe Met Pro Ala Ala Gln Leu
```

|  |  | 305 |  |  |  | 310 |  |  |  | 315 |  |  |  | 320 |

```
        Pro Glu Leu Pro Asp Val Arg Leu Pro Thr Asn Lys Asp Val Cys Asp
                        325                 330                 335

        Pro Gly Asn Thr Lys Val Met Asp Lys Tyr Thr Phe Glu Leu Ser Arg
                    340                 345                 350

        Arg Thr His Leu Pro Glu Val Phe Leu Ser Lys Val Leu Glu Pro Thr
                355                 360                 365

        Leu Lys Ser Leu Gly Glu Cys Cys Asp Val Glu Asp Ser Thr Thr Cys
            370                 375                 380

        Phe Asn Ala Lys Gly Pro Leu Leu Lys Lys Glu Leu Ser Ser Phe Ile
        385                 390                 395                 400

        Asp Lys Gly Gln Glu Leu Cys Ala Asp Tyr Ser Glu Asn Thr Phe Thr
                    405                 410                 415

        Glu Tyr Lys Lys Lys Leu Ala Glu Arg Leu Lys Ala Lys Leu Pro Glu
                420                 425                 430

        Ala Thr Pro Thr Glu Leu Ala Lys Leu Val Asn Lys Arg Ser Asp Phe
                435                 440                 445

        Ala Ser Asn Cys Cys Ser Ile Asn Ser Pro Pro Leu Tyr Cys Asp Ser
            450                 455                 460

        Glu Ile Asp Ala Glu Leu Lys Asn Ile Leu
        465                 470
```

## Description

The present invention discloses a diagnosis and/or progression method of a disease as defined in the claims based on the detection and quantification of the expression levels of biomarkers preferably selected from the group comprising gelsolin isoforms (SEQ. ID 1 and 2) and Vitamin D binding protein isoforms (SEQ. ID 3 and 4). Said method is applied to central nervous system diseases, preferably to Multiple Sclerosis.

State of the art

The inflammatory and degenerative diseases affecting the central nervous system comprise a wide range of diseases for which very often, to date, no efficient treatment therapy is available. For many of these, a diagnosis is also particularly difficult, but when the disease is full blown, when the therapeutic symptomatic approaches are most of times less efficient than the same therapies introduced in the onset stage. A further major limitation is the difficulty in monitoring the disease progression, which progression often has a wide heterogeneity in the various afflicted subjects; this difficulty translates into the impossibility of monitoring in an objective manner the effectiveness of the therapeutic approach in the particular individual.

Particular important among those diseases affecting the central nervous system is Multiple Sclerosis (MS). MS is a demyelinating chronic inflammatory disease, with autoimmune pathogenesis, affecting the central nervous system (brain and spinal cord).

The natural history of the disease is heterogeneous. Onset symptoms may occur, individually or in combination, in an acute form that completely or partially relapse, or in a slowly progressive form. It may mainly involve the sensory-motor, visual systems, and the cerebellar/vestibular system (Ghezzi et al., 1980). Usually, at a variable time interval, re-exacerbation phases may occur, combining the most various combinations of symptoms: the most recurrent remain those of the onset, with the particularity that, upon time, outcomes tend to become fixed, showing other disorders besides to the initial ones. In the most advanced phases, the disease progression becomes stable, compromising a number of important functional systems. The wide variability in the symptoms characterizing it is due to a degeneration process of myelin, the protective sheath covering and isolating the nervous fibers and allowing the quick and integral transmission of signals thereby. In the course of the disease, the destruction of myelin sheaths causes the block or slowing of the pulses going from the central nervous system to different areas in the body, and vice versa. The areas in which myelin was damaged are referred also to as plaques; hence the definition of plaque sclerosis.

The disease often affects subjects aged 20 - 40 years, even if the onset of the disease in children - teenagers (before 15 years) and after 50 years is getting more and more frequent. The prevalence for women is clear, with a F/M ratio of 2:1; prevalence data in Italy show a ratio of 50/90 for every 100,000 population.

MS onset is determined by a recurrent, immune-based inflammatory process, damaging myelin, and secondly to the damage to the latter, also to the axons contained therein, which irreversibly deteriorate, forming a base for the progressive permanent disability. Such demyelination phenomena are diffused in the white matter of the Central Nervous System (CNS), encephalon, and marrow, and give rise to a variety of signs and symptoms reflecting the different localization of brain, marrow, or optical nerve focal lesions.

The symptoms of the disease depend on the localization of the demyelination areas, which represent the most important histo-pathologic lesion in MS; however, the appearance of symptoms can be caused by both the edema and the action of toxic inflammatory mediators, and the axonal loss. The progressive damage to the axons, in cases with a chronic course, leads to an extensive degeneration and cerebral atrophy, which seems to be strongly related to permanent neurological deficits, in a higher extent to demyelination (Poser et al., 1985).

Etiology of the disease is still unknown, while its pathogenesis seems to be related to a combination of various factors. MS can be considered as a disease that is paradigmatic of a set of pathological conditions currently known as multifactorial diseases, where genetic and environmental factors interact in a complex manner.

In 1996 the definitions for the different clinical courses of MS were standardized:

- relapsing - remitting MS (MSRR),
- secondary progressive MS (MSSP),
- primary progressive MS (MSPP).

MSRR is characteristics of patients having an initial exacerbation, followed by a complete or partial recovery. Although approximately 85% of patients with MS follows this disease course from its onset, 50% within ten years, and 90% after 25 years develops a gradual progression of disability that can be accompanied by relapses or not; in such a case, the disease is referred to as secondary progressive MS (MSSP). 10-15% of patients shows a gradual progression of the disability from the onset, which is not accompanied by exacerbations: this is the primary progressive (MSPP) form. Finally, a new term, progressive relapsing MS (MSPR), was introduced to describe patients with a disease course that is progressive from the onset, subsequently accompanied by one or more relapses with a worsening in the progression in the intervals between relapses (Lublin and Reindgold, 1996).

Predicting the clinical course of the disease is difficult, considered the large interindividual variability of the clinical course (from benign, even asymptomatic cases, to clinical forms with more severe signs).

To date, there is still no specific therapy for MS. Pharmacological treatments target acute episodes, the prevention of relapses, and a general improvement of symptoms.

The diagnosis of MS is not easy, above all because, upon its onset, the disease does not show characterizing symptoms; instead, in most of cases they appear suddenly and are generally non-specific, and could also be the result of other, less severe diseases. To reach a MS diagnosis, to date, no test is available, which is able to confirm in a definite and indisputable manner the diagnosis of MS; however, the latter is given by a clinician based on three elements: i) thesymptoms complained of by the patient; ii) neurological examination; iii) positive outcome of some instrumental examinations, among which mainly nuclear magnetic resonance (NMR). NMR is a sensitive and specific examination that is able to determine the cerebral and medullary lesions responsible for the various neurological signs and symptoms of MS. However, the appearance of the lesions is a late event occurring after the pathogenetic molecular events.

The need to have an early diagnosis method is strongly felt, since it would allow a timely ad correct therapeutical approach. A number of studies showed that exactly an early pharmacological treatment allows reducing by 50% the risk of developing this disease, besides decreasing the aggressiveness thereof and slowing its progression (Freedman, 2011). Anticipating, predicting and blocking are the three keywords to avoid the progression of the disease.

Currently, no biochemical - molecular tool is available, allowing the physicians early identifying MS patients and recognizing their clinical type. In the practice, only the presence in the cerebrospinal fluid of oligoclonal Immunoglobulins IgG is usually used as a biochemical tool for supporting the clinical diagnosis of Multiple Sclerosis.

The biomedical research on MS has, among its priorities, the identification of specific biomarkers associated to the disease and the different manifestations thereof. In literature, the involvement in MS of gelsolin is disclosed, which is a multifunctional protein capable of bonding actin; in particular, decreases in the levels of gelsolin in the cerebrospinal fluid (CSF) of MS patients were observed (Kulakowska et al., 2008, 2010). In pediatric MS patients, instead, an increase in the levels of gelsolin and also of Vitamin D binding protein (DBP) was shown (Rithidech et al., 2009). Further studies pointed out that DBP levels in the CSF are lower during remission stages, to then increase in the secondary progressive stage (Disanto et al., 2011). PCT/SE2006/000866 proposes to assess the levels of phosphorylated DBP as a diagnostic tool to distinguish MS patients affected by the RR form, from those with a SP form.

In spite of the large number of studies about it, to date, biomarkers that are not only qualitative, but also quantitative, associated to MS and that can be used clinically are not available. In particular, to date, biomarkers capable of distinguishing, upon the diagnosis, those patients who will develop a full-blown form of MS from those who will develop other neurological diseases are not available, and markers for the progression of the disease that may help the physician in prescribing a more suitable pharmacological treatment are not available either. A not timely or not suitable pharmacological intervention is one of the reasons for which the available treatments show a effectiveness that is limited and variable from patient to patient. Moreover, it shall be noticed that, in the absence of efficient therapies, a prompt diagnosis would allow starting the treatments in advance, then to prolong life expectancy and quality of the patient (Lacomblez et al., 1996). Furthermore, the absence of biomarkers does not allow an efficient classification on a molecular basis of the different phenotypes of MS, which is a complex multifactorial disease that probably encompasses within its clinical definition various neurological disorders (Shaw and Williams, 2000; Bowser et al., 2006). On the other hand, the absence of specific biomarkers, reflects poor. knowledge of the molecular mechanisms involved in the onset and development of MS, with the result that, in testing new compounds for therapeutic action, it is not possible to obtain a quantitative indication of the effectiveness thereof. To this effect, the discovery of new biomarkers may concur to the understanding of the disease mechanisms, and therefore to the development of possible new therapeutic targets.

Between the bodily fluids that can be analyzed in order to identify MS biomarkers, CSF represent a unique source of information relative to the disease and its pathogenetic mechanisms, since it is secreted by CNS structures and contains peptides, proteolytic fragments, and antibodies that may reflect the presence and progression of the disease. Therefore, the discovery of new biomolecules in the CSF of affected patients may improve the diagnostic discrimination within the heterogeneous extent of the disease. In particular, the identification of biomarkers capable of distinguishing clinically relevant MS subgroups would represent a powerful, promising tool to determine prognostic factors, to monitor the clinical course in different MS phenotypes, as well as to identify therapeutic targets in function of a customized cure.

WO02059604 discloses inter alia that the levels of gelsolin are correlated with the onset and development of Multiple Sclerosis.

Ottervald J et al., Journal of Proteomics, 2010 vol. 73, pages 1117-1132 and Disanto et al., Journal of Neurology 2010, vol. 258, pages 353-358 disclose inter alia that the levels of Vitamin D Binding Protein are correlated with the onset and development of Multiple Sclerosis.

To the light of what has been pointed out above, the urgent need to find MS-specific biomarkers useful for the diagnosis, formulation of the prognosis, and the monitoring of the disease progression in its different definitions will be apparent. Therefore, the object of the present invention is to provide a method for diagnosis, progression, and prognosis of MS as defined in the claims based on the individuation and identification of specific biomarkers. Said biomarkers turn out to be useful not only for MS; but also for other inflammatory and degenerative diseases affecting the central nervous system.

Description of the invention

By a proteomic analysis carried out on biologic fluid samples obtained by subjects affected by inflammatory and degenerative diseases of the central nervous system, markers were shown, the trend of which is surprisingly quantitatively related to the disease.

Description of the Figures

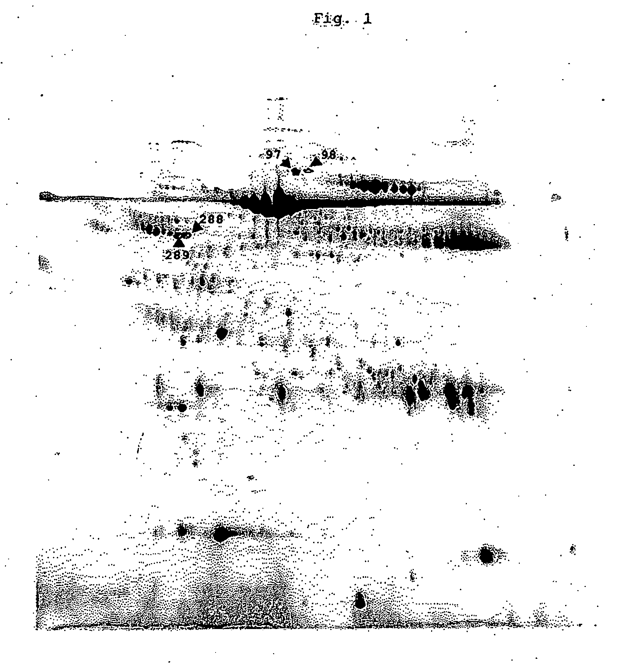

Fig. 1: bidimensional gel, two spots are shown, relative to gelsolin isoforms precursors (ID 97, 98) and two spots relative to two DBP isoforms (ID 288, 289).
Fig. 2: dendrogram resulting from the cluster analysis performed on the average values of the percent volumes (Vol%) of the most representative spots of the analyzed gel population (250 spots), two groups are shown, A and B;
Fig. 3: dendrogram resulting from the cluster analysis performed by selecting the set of biomarkers included in the panel 1 described in Table 3, two groups are shown, A1 and B1;
Fig. 4: trend of the average Vol% $\pm$ SD of the spots ID 97, 98, and 289 in the two clusters A1 and B1;
Fig. 5: levels of the average Vol% of the spots ID 288 and 289 in the individual patients included in the study;
Fig. 6: dendrogram obtained from the cluster analysis based on the Vol% of the spots ID 288 and 289, two groups are shown, G and Z.
Fig. 7: combination of the values of the Vol% of the spots ID 288 and 289 in the groups G and Z.

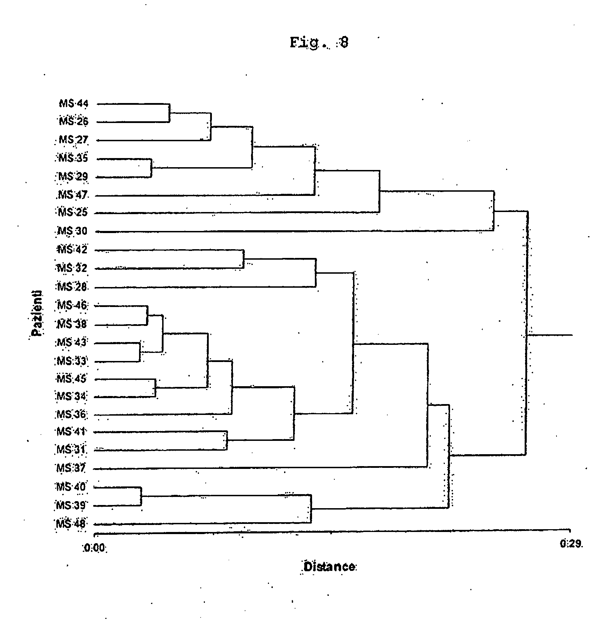

Fig. 8: dendrogram resulting from the cluster analysis based on the Vol% of the spots ID 97, 98, 288 and 289.

Detailed description of the invention

Unless explicitly indicated otherwise, the following terms used in the present application are meant in the meaning indicated herein below:

"percentage of identity" and "% of identity" between two amino acid (peptides) or nucleic acid sequences (nucleotides): percentage of amino acid or nucleotide acid residues that are identical in corresponding positions in the two sequences optimally aligned. In order to determine the "percentage of identity" of the two amino acid or nucleic acid sequences, the sequences are aligned together; to get an optimal comparison, interruptions can be introduced in the sequences (i.e., deletions or insertions - which can optionally be located also at the end of the sequences) (gap). The amino acid and nucleotidic acid residues in corresponding positions are then compared. When a position in the first sequence is occupied by the same amino acid or nucleotide residue that occupy the corresponding position in the second sequence, the molecules are identical in that position. The percentage of identity between two sequences is as a function of the number of identical positions shared by the sequences [i.e., % of identity = (number of the identical positions / total number of the positions) X 100]. According to an advantageous embodiment, the sequences have the same length. Advantageously, the compared sequences do not have interruptions (or insertions).

The percentage of identity can be obtained by using mathematical algorithms. A non-limiting example of a mathematical algorithm used for the comparison of two sequences is the Karlin and Altschul algorithm [Proc. Natl. Acad. Sci. USA 87 (1990) 2264-2268] modified by Karlin and Altschul [Proc. Natl. Acad. Sci. USA 90 (1993) 5873-5877]. Such algorithm is incorporated in the BLASTn and BLASTp programs by Altschul [Altschul, et al., J. Mol. Biol. 215 (1990) 403-410].

In order to obtain alignments even in the presence of one or more interruptions (or insertions), it is possible to use methods allocating a relatively high penalty to each interruption (or insertion), and a lower penalty for each additional amino acid or nucleotide residue in the interruption (such additional amino acid or nucleotide residue is defined as an extension of the interruption). It shall be apparent that high penalties will determine optimized alignments with a lower number of interruptions. When using the BLAST programs, the BLOSUM62 matrix is typically employed.

An advantageous, non-limiting example of a program to implement an optimal alignment is the GCG Winsconsin Bestfit package (University of Winsconsin, USA; Devereux et al., 1984, Nucleic Acids Research 12:387). Also in this case, the default parameters are used that, for an amino acid sequence, provide for a penalty of -12 for an interruption, and a penalty of -4 for each extension.

By "percentage of homology" and "% of homology" between two amino acid or nucleic acid sequences is meant the percentage of homologous amino acid or nucleotide residues in corresponding positions in the two optimally aligned sequences. The percentage of homology between two sequences is determined in a manner that is substantially identical to what has been described above for determining the percentage of identity, except in that, in the computation, also the homologous positions are considered, not only the identical positions. As regards a nucleotide sequence, two homologous positions have two different nucleotides, but which within their own codon lead to the codification of a same amino acid. As regards an amino acid sequence, two homologous positions have two homologous amino acids, i.e., amino acids that are provided with similar chemical-physical properties, for example, amino acids belonging to the same groups, such as: aromatic (Phe, Trp, Tyr), acid (Glu, Asp), polar (Gln, Asn), basic (Lys, Arg, His), aliphatic (Ala, Leu, Ile, Val) groups, with a hydroxyl group (Ser, Thr), with a short side chain (Gly, Ala, Ser, Thr, Met). It is expected that substitutions between such homologous amino acids do not alter the protein phenotype (conservative substitutions of amino acids). Specific examples of conservative substitutions are known in this technical field, and are described in literature [e.g., Bowie et al., Science, 247:1306-1310 (1990)].

"Corresponding position"- a position in a sequence of a polypeptide or nucleic acids corresponding to (facing), following an alignment, a determined position of a reference sequence.

By "level" is meant the level of expression of a protein in the sample, as measured with one of the techniques as described herein and/or known to those skilled in the art.

It is the object of the present disclosure the identification of polypeptides that are present in biologic fluids of patients affected from autoimmune neurological disease, degenerative diseases of the central nervous system, inflammatory diseases of the central nervous system, the expression levels of which can be related to well-defined clinical pictures and pathologic phenotypes. Preferably, said diseases will be selected from Multiple Sclerosis, the neuromyelitis optica (Devich's disease), encephalites associated with auto-antibodies, among which encephalites associated with anti-NMDA antibodies and anti-GAD antibodies (Stiff Person syndrome), paraneoplastic syndromes of the central nervous system with auto-antibodies (for example anti-Tr antibodies; Alzheimer's disease, Parkinson's disease; viral or bacterial central nervous system infections (encephalites and meningites), vasculitides, demyelinating diseases. In the invention the disease is Multiple Sclerosis. Said polypeptides were identified by known proteomics techniques, to be then identified by means of mass spectrometry techniques. The identification of said peptides is set forth in the following Table 1.

Table 1: Mass spectrometry identification of some of the selected spots.

| Spot ID | LC-MS/MS identification | Accession No. | Peptide No. | AA coverage (%) | Sequence coverage | Theoretical MW (Das)/pI | Mascot score |
|---|---|---|---|---|---|---|---|
| 70 | Complement factor B | gi 291922 | 10 | 18 | 51-696 | 85.4/6.5 | 514 |
| 72 | Complement factor B | gi 291922 | 12 | 18 | 183-707 | 85.4/6.5 | 222 |
| 97 | Gelsolin isoform precursor | gi4504165 | 21 | 37 | 33-748 | 85.6/5.9 | 415 |
| 98 | Gelsolin isoform precursor | gi4504165 | 6 | 10 | 148-738 | 85.6/5.9 | 117 |
| 99 | Afamin precursor | gi 4501987 | 8 | 14 | 89-453 | 69/5.6 | 52 |
| 101 | Serum transferrin | gi 110590597 | 6 | 10 | 25-508 | 74.6/6.6 | 159 |
| 102 | Serum transferrin | gi 110590597 | 18 | 29 | 25-674 | 74.6/6.6 | 346 |
| 117 | Glycoprotein alpha 1-β | gi 69990 | 3 | 6 | 86-415 | 51.9/5.6 | 77 |
| 227 | Albumin fragment | gi 28592 | 3 | 5 | 427-581 | 69.3/6 | 56 |
| 288 | Vitamin D-binding protein | gi 181482 | 13 | 30 | 52-440 | 53.0/5.4 | 291 |

(continued)

| Spot ID | LC-MS/MS identification | Accession No. | Peptide No. | AA coverage (%) | Sequence coverage | Theoretical MW (Das)/pI | Mascot score |
|---|---|---|---|---|---|---|---|
| 289 | Vitamin D-binding protein | gi 181482 | 20 | 56 | 52-471 | 52.9/5.4 | 480 |
| 469 | Apolipoprotein E | gi 178853 | 14 | 50 | 20-317 | 36.1/5.8 | 348 |

In particular, it is pointed out that the two polypeptides ID 288 and 289 represent modified DBP isoforms. Considering the migration of the two spots, since an increase in glycosylation is known to result in a migration to a more acidic position, the spot ID 289, being to the left side compared to the spot ID 288, would be a glycosylated isoform of DBP.

The biomarkers identified in the present invention can be detected and quantified by techniques that are known to those skilled in the art for the analysis of proteins, selected preferably from the group comprising Western-Blot, 2DE gel electrophoresis, ELISA *(Enzyme-Linked Immunosorbent assay)*, RIA (*Radioimmunoassay*), Competitive EIA (*Competitive Enzyme Immunoassay*), DAS-ELISA (*Double Antibody Sandwich-ELISA*), other immunocytochemical or immunohistochemical techniques. Said techniques will be combined, as necessary and as well known to those skilled in the art, with HPLC or mass spectrometry. In a preferred embodiment, said assessments are carried out by 2DE gel electrophoresis.

In particular, it has been pointed out that the levels of ID 97, ID 98, and ID 289 are increased in those patients affected by a pathological form that will develop into a more severe course. On the other hand, ID 288 is increased in patients affected by a pathological form with a slower, less aggressive progression. Surprisingly, the present analysis has also shown that the values of the ID 288 and ID 289 levels are inversely related. In particular, the values of the ID 289 and ID 288 levels were combined together by means of the linear functions f1 and f2:

$$f1 = 33.813 * (ID\ 288\ level) + 48.942 * (ID\ 289\ level) - 7.155;$$

$$f2 = 103.508 * (ID\ 288\ level) + 42.871 * (ID\ 289\ level) - 15.248.$$

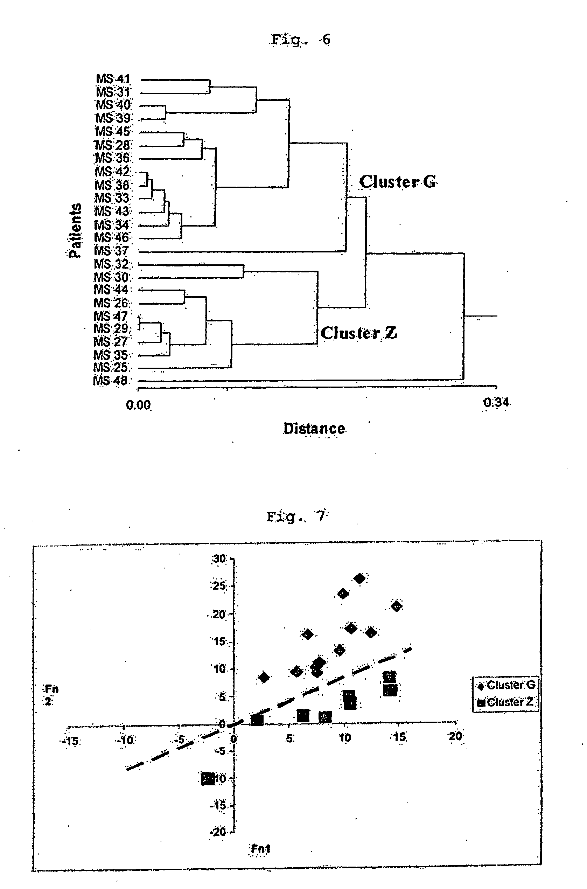

by illustrating in a graph the values that the two functions take based on the levels measured in each of the analyzed samples, a good separation of the two populations, clusters G and Z, (Fig. 7) has been observed, and the straight line capable of separating them was described by the function $y = 1.5629x - 1.7155$. Values exceeding the straight line defined by the function $y = 1.5629x - 1.7155$ are associated with a less severe progression of the disease (cluster G); those values that are below the same straight line are associated to a more severe progression (cluster Z). Therefore, the function $y = 1.5629x - 1.7155$ turns out to be able to stratify the population of samples based on the severity of the disease progression.

It is claimed herein a method of diagnosis and progression of Multiple Sclerosis comprising:

i. measuring the ID 289 (SEQ. ID 4) and ID 288 (SEQ. ID 3) level in a sample C;
ii. obtaining the coordinates f1 and f2, where f1 = 33.813 * ID 288 level + 48.942 * ID 289 level - 7.155 and f2 = 103.508 * ID 288 level + 42.871 * ID 289 level - 15.248;
iii. positioning in the Cartesian plane the point C(f1, f2) and assessing the position thereof relative to the straight line $y = 1.5629x - 1$;
iv. where C(f1, f2) > $y = 1.5629x - 1$, the sample C will be assigned to the MSSP or MSPP group; where C(f1,f2) < $y = 1.5629x - 1$, the sample C will be assigned to the MSRR group.

Where said assessments are carried out by 2DE gel electrophoresis, said levels are related to the Vol% of the spots measured for ID 288 and ID289, respectively.

Furthermore, it has been noticed that the Vol% values of the spots ID97 and ID98 are capable of distinguishing between MSSP or MSPP and MSRR. A method comprising the assessment of the levels of the gelsolin isoforms ID97 (SEQ. ID 1) and/or ID98 (SEQ. ID 2) in the sample and the comparison of the same levels assessed in a subject affected by MSRR is here described. Said levels of the gelsolin isoforms ID97 (SEQ. ID 1) and ID98 (SEQ. ID 2) are assessed by a method capable of distinguishing between the two isoforms, or by a method capable of assessing the two isoforms in a combined manner or, alternatively, of assessing also a single isoform. By G, the value of said levels of the gelsolin isoforms is herein defined, whether it is obtained from the sum of the isoforms ID97 and ID98 assessed independently, or from the same isoforms assessed in a combined manner or, alternatively, only of the isoform ID 97 or only of the isoform ID98. Where the value measured in the sample was increased in a statistically significant manner with respect to the value measured in the MSRR subject, said sample will be assigned to the MSSP - MSPP group. Where the value measured in the sample does not depart in a statistically significant manner from the value measured in the MSRR subject, said sample will be assigned to the MSRR group.

A method of assessment of the progression of Multiple Sclerosis is herein described, comprising:

i. measuring the ID 97 (SEQ. ID 1) and/or ID 98 (SEQ. ID 2) level in a sample C and in a control MSRR;

ii. obtaining the value G = ID 97 level + ID 98 level, or G = ID 97 level or G = ID 98 level in the sample $G_c$ and in the control $G_{CtrlMSRR}$;

iii. where $G_C > G_{CtrlMSRR}$ in a statistically significant manner, assigning the sample C to the MSSP or MSPP group; where $G_C = G_{Ctrl}$, the sample C will be assigned to the group MSRR.

In a further embodiment, the method of diagnosis and progression claimed herein comprises:

i. measuring the ID 97 (SEQ. ID 1) and/or ID 98 (SEQ. ID 2) level in a sample C and in a control MSRR, and measuring the ID 289 (SEQ. ID 4) and ID 288 (SEQ. ID 3) level in the sample C;

ii. obtaining the coordinates f1 and f2, where f1= 33.813 * ID 288 level + 48.942 * ID 289 level - 7.155 and f2= 103.508 * ID 288 level + 42.871 * ID 289 level - 15.248;

iii. positioning in the Cartesian plane the point C(f1, f2) and assessing the position thereof relative to the straight line y = 1.5629x - 1;

iv. obtaining the value G = ID 97 level + ID 98 level, or G = ID 97 level or G = ID 98 level in the sample ($G_C$) and in the control ($G_{CtrlMSRR}$);

v. where C(f1,f2) > y = 1.5629x - 1 and Gc > $G_{Ctrl}$ in a statistically significant manner, assigning the sample C to the MSSP or MSPP group ; where C(f1, f2) < y = 1.5629x - 1 and $G_C = G_{CtrlMSRR}$, assigning the sample C to the MSRR group.

It is a further object of the present disclosure a method of diagnosis and progression of the disease, comprising obtaining the coordinates f1 and f2 as defined above and/or of the value G as defined above and the combination of the assessment of C(f1, f2) and/or G with the assessment of further biomarkers selected, by way of illustrative, non-limiting example, in the group comprising: complement factor C3, complement factor C4, beta-2-microglobulin, Clusterin, Prostaglandin H2 D isomerase, Haptoglobin, Immunoglobulins, Apolipoprotein E, Beta fibrinogen, alpha 2 macroglobulin, Fetuin A, serin protease inhibitor, Tansthyretin, Albumin, Transferrin, Apolipoprotein D, Retinol binding protein, Apolipoprotein A4, *SPARC-like* protein, Autotaxin t, Pigment epithelium derived factor, Angiotensinogen, Chromogranin A, Tuberous sclerosis complex 2, Cystatin C, Ceruloplasmin, Superoxide dismutase 1, Actin, Beta V Spectrin, Cartilage acidic protein 1, Fibulin 1, Calsyntenin 3, Contactin 1, Xantin dehydrogenase/oxidase, RNA binding motif protein 7, Ribonuclease 1, neuroendocrine protein 7 B2, alpha 1 Antitrypsin, alpha 1 Antichymotrypsin, Kallikrein 6, EGF-containing fibulin-like extracellular matrix protein 1, Plasminogen, Antithrombin III, Dickkofp relata protein 3, neuronal pentraxin receptor, Tetranectin, so that the combination of C(f1,f2) and/or G with one or more of said biomarkers results in obtaining enhanced predictive statistical power. It shall be apparent that such combinations fall within the object and scope of the instant patent, as well as any other combinations of C(f1,f2) and/or G with other biomarkers and/or physiological and/or diagnostic markers.

It is further described a kit for implementing the methods described above, the kit comprising two antibodies capable of specifically recognizing the two isoforms of Vitamin D Binding protein SEQ. ID 3 and SEQ. ID 4, and/or one or two antibodies capable of recognizing in a combined or selective manner the gelsolin isoforms SEQ. ID 1 and SEQ. ID 2 and, optionally, one or more antibodies capable of recognizing one or more of the selected biomarkers, by way of illustrative, non-limiting example, in the group comprising: complement factor C3, complement factor C4, beta-2-microglobulin, Clusterin, Prostaglandin H2 D isomerase, Haptoglobin, Immunoglobulins, Apolipoprotein E, Beta fibrinogen, alpha 2 macroglobulin, Fetuin A, serin protease inhibitor, Tansthyretin, Albumin, Transferrin, Apolipoprotein D, Retinol binding protein, Apolipoprotein A4, SPARC-like protein, Autotaxin t, Pigment epithelium derived factor, Angiotensinogen, Chromogranin A, Tuberous sclerosis complex 2, Cystatin C, Ceruloplasmin, Superoxide dismutase 1, Actin, Beta V Spectrin, Cartilage acidic protein 1, Fibulin 1, Calsyntenin 3, Contactin 1, Xantin dehydrogenase/oxidase, RNA binding motif protein 7, Ribonuclease 1, neuroendocrine protein 7 B2, alpha 1 Antitrypsin, alpha 1 Antichymotrypsin, Kallikrein 6, EGF-containing fibulin-like extracellular matrix protein 1, Plasminogen, Antithrombin III, Dickkofp relata protein 3, neuronal pentraxin receptor, Tetranectin. Said kit may comprise also reagents for the detection of said antibodies bound to said proteins.

The method claimed herein, in its various embodiments, offers an objectivity that would not be otherwise obtained with the clinical methods currently in use for the diagnosis, progression, and prognosis of the disease, the method being claimed herein as exclusively related to a molecular aspect of the disease and the measurement of quantitative parameters.

It is further described the induction of the isoform of the Vitamin D binding protein SEQ. ID4, such induction being obtained by acting on the metabolic processes resulting in the post-translational modifications observed in the spot ID289 with respect to the spot ID 288. In particular, the induction of the enzyme sialidase is herein described, which is obtained by an increased synthesis, a decreased catabolism and/or, a provision of co-factors. In a preferred embodiment, said induction of the enzyme sialidase is obtained by a recombinant vector selected from the vectors that are integrated or not integrated in the genome, characterized in that said recombinant vector comprises a gene coding for sialidase or an homologous thereof, or contains an element capable of acting at the level of the endogenous promoter of said sialidase by activating it. Furthermore, the use of inducers of galactosidases is described, in particular of Beta galactosidase and N acetyl galactosaminidase. Said inducers are preferably selected from the group comprising isopropyl-b-D-thiogalactopyranoside (IPGT) and lactose. In a further embodiment, an inhibition of the enzyme glucosyltransferase is claimed, obtained preferably with Interferon.

A composition is also described, comprising one or more of the compounds listed above and pharmacologically acceptable excipients for use in the treatment of MS.

<u>Examples</u>

**Example 1: Sample collection**

CSF samples of a homogeneous population of 24 women affected by Multiple Sclerosis were collected. The clinical data of the patients enrolled for this study are described in detail in the following Table 2.

Table 2: Clinical data of the patients.MS enrolled in the instant study.

| RRMS patients | OCB | CSF protein (mg/dl) | Serum protein (mg/dl) | CSF IgG (mg/dl) | Serum IgG | CSF Albumin (mg/dl) | IgG Index | Cells/μl |
|---|---|---|---|---|---|---|---|---|
| MS 25 | tras | 20 | 683 | 0.585 | 443.7 | 8.7 | 0.44 | 3 |
| MS 26 | pos | 34 | 1190 | 3.52 | 345.6 | 15.7 | 0.65 | 5 |
| MS 27 | pos++ | 36 | 1467 | 11.5 | 378.5 | 14.4 | 2.1 | 28 |
| MS 28 | pos++ | 72 | 909 | 7.32 | 382.7 | x | 0.5 | 20 |
| MS 29 | pos++ | 30 | 1562 | 5.08 | 494.5 | 16.9 | 1 | 16 |
| MS 30 | pos++ | 22 | 1475 | 3.72 | 436.4 | 9.3 | 1.18 | 20 |
| MS 31 | pos++ | 35 | 1170 | 18.7 | 548.4 | 13.4 | 6.5 | 45 |
| MS 32 | pos++ | 36 | 634 | 3.38 | 360.1 | 13.7 | 1.4 | 8 |
| MS 33 | pos++ | 35 | 790 | 6.21 | 443.8 | 24.1 | 1.4 | 38 |
| MS 34 | pos++ | 33 | 920 | 7.22 | 448.6 | 23.6 | 1.5 | 16 |
| MS 35 | pos | 25 | 892 | 2.75 | 437.8 | 21 | 0.6 | 3 |
| MS 36 | pos++ | 38 | 1002 | 7.67 | 325.8 | 17.4 | 1.4 | 5 |
| MS 37 | pos++ | 29 | 1072 | 3.39 | 338.7 | 19.2 | 0.6 | 3 |
| MS 38 | pos++ | 21 | 988 | 3.32 | 362.2 | 12.6 | 0.1 | 8 |
| MS 39 | pos++ | 59 | 903 | 4.96 | 349 | 39.1 | 0.5 | 3 |
| MS 40 | tras | 49 | 1133 | 3.03 | 522.7 | 22.2 | 0.63 | 3 |
| MS 41 | neg | 36 | 1241 | 3.51 | 297.5 | 23.5 | 0.4 | 3 |
| MS 42 | neg | 32 | 1023 | 1.62 | 397.8 | 23.5 | 0.3 | 2 |
| MS 43 | pos++ | 46 | 1463 | 6.74 | 383.8 | 27.6 | 0.6 | 8 |
| MS 44 | pos++ | 40 | 1310 | 2.27 | 372.3 | 33.5 | 0.2 | 2 |
| MS 45 | neg | 43 | 1052 | 3.66 | 402 | 33.1 | 0.42 | 2 |
| MS 46 | pos++ | 35 | 955 | 4.7 | 346.1 | 20.7 | 0.82 | 8 |

(continued)

| RRMS patients | OCB | CSF protein (mg/dl) | Serum protein (mg/dl) | CSF IgG (mg/dl) | Serum IgG | CSF Albumin (mg/dl) | IgG Index | Cells/μl |
|---|---|---|---|---|---|---|---|---|
| MS 47 | pos++ | 29 | 1103 | 4.39 | 432.7 | 17.3 | 1 | 6 |
| MS 48 | pos | 31 | 644 | 1.61 | 334.5 | 23.6 | 0.4 | 2 |

Legenda:
CSF: *Cerebro Spinal Fluid*
OCB: *Oligo Clonal Band*

Upon the collection, said patients were affected by a RR form of Multiple Sclerosis according to the clinical criteria established by Mc Donald's (2001).

The average age of the subjects included in the study upon the liquor collection was about 36 years: the youngest subject was 17 years old, while the oldest one was 61 years old.

The liquor samples were collected in the remitting phase, and all the subjects, except for the patients MS 27, MS 29, MS 35, MS 25, and MS 26, were not undergoing a pharmacological treatment.

The lumber puncture for CSF withdrawal was performed between the fourth and fifth intervertebral disk space according to a procedure that allowed withdrawing a liquor volume of 20 mL. The CSF samples obtained from each subject were collected in propylene tubes in the presence of protease inhibitors, centrifuged at 1,000 x g at 4° C for 10 minutes, and finally stored at -80° C, until the time of analysis.

In order to allow the protein extraction, the biological samples were subjected to a process of concentration and elimination of salts by means of special devices, having a 5kDa filter (Ultrafree Millipore, Bedford, USA) according to the procedures indicated in the use manual. Albumin was not removed from the samples, not only because albumin is a transport protein that can bind markers of interest, but also because modified forms of albumin differentially present in the disease of interest may play a role for diagnostic purposes. Therefore, the removal of albumin may lead to the loss of useful biomarkers.

**Example 2: Follow up**

The patients were monitored in the two years following the collection, except for the patients MS 40 and MS 43.

**Example 3: 2DE and statistical analysis**

On the samples prepared as in the Example 1, a 2DE experiment was carried out at least in duplicate, according to the procedure described by Robotti et al. 2010, with some modifications. For each sample, 100 μg total protein was heated for 5 min at 95° C in the presence of 10 μl sodium dodecylsulfate (SDS) at 5% (w/v) and dithiothreitol (DTT) at 2.5% (w/v), and then diluted to 330 μl with a buffer containing urea (7 M), thiourea (2 M), 3[(3-Colamidopropyl)dimethylammonium]-propansulfonic acid (CHAPS) at 4% (w/v), IPG 3-10NL buffer at 0.5% (v/v)(GE Healthcare, Uppsala, Sweden), and traces of bromophenol blue. The sample was then loaded on 18 cm non-linear strips for isoelectrofocusing (IEF) from IPG 3-10 (GE Healthcare) with rehydratation in gel (2 h at 0 V, and 12 h at 30 V). A IEF was carried out at 20° C with IPGphor apparatus (GE Healthcare), according to the following protocol: 500 V at 500 V/h, 1.000 V at 1.000 V/h with linear gradient; 8,000 V at 13,500 V/h with linear gradient; 8,000 V at 72,000 V/h. Before the denaturating electrophoresis in gel of polyacrylamide-SDS (SDS-PAGE), the IPG strips were equilibrates twice for 15 min in Tris-HCl buffer (50 mM) at pH 8.8, urea (6 M), 30% glycerol (v/v), SDS al 2% (w/v) and traces bromophenol blue, containing 1% DTT (w/v) for the first passage and 2.5% iodoacetamide (w/v) for the second passage. This was then followed by SDS-PAGE in a 12.5% gel (1.5 mm thickness), according to the Laemmli protocol [1970], but without stacking gel, by using a Hoefer SE600 apparatus (GE Healthcare). The second dimension was stroke at 60 ma/gel at 16° C, and it was stopped when the front part of the bromophenol blue reached the lower end of the gel. For the calibration of the molecular weights (MW) and of the isoelectric points (pI) proteins having a MW ranging between 15 and 100 kDa and with a pI ranging between 4.5 and 8.5 were used as standard. The gels were stained with Sypro Ruby (Molecular Probes Inc, Eugene,OR). After staining, digital images of the gel were taken by using a ProXPRESS 2D CCD camera (Perkin Elmer). The images were analyzed by the ImageMaster 2D Platinum 5.0 software (GE Healthcare). A "detection" of the spots of each image was performed, then only one reference gel was selected, i.e., the gel containing the highest number of correctly focused spots. Each gel was then virtually overlapped ("matching") on the reference gel. The matching was automatically performed by the software; subsequently, it was manually corrected. For each spot, Vol% was calculated as the integral of the volume of each spot stained with Sypro (area of the spot multiplied by its intensity) normalized on the sum of the volumes of all the spots present in the gel. The data relative to the most representative % volumes of the spots, i.e., those present at least in 80% of the analyzed population (250 spots) were exported in an Excel worksheet, and for each of them the average Vol% value obtained from at least two technical replicates was calculated. These data have been subjected to a cluster analysis by using the StatistiXL software version 1.x (CustomCD, http://www.statisticxl.com). The used approach provided for the use of a hierarchical cluster analysis, in which a hierarchy of partitions was built, which were characterized by a decreasing/increasing number of groups, visible by a tree representation (dendrogram), in which the grouping/division steps of the groups are represented. The Euclidean distance was considered as the similarity measure.

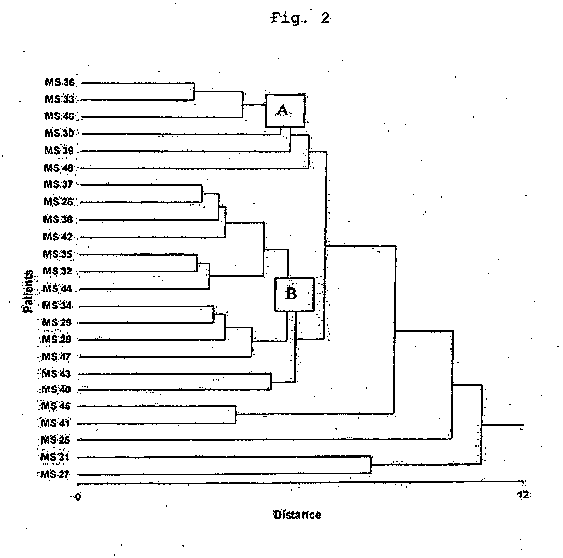

From this cluster analysis, two main subgroups of patients were identified (Cluster A and Cluster B), and 5 outlier subjects (Fig. 2). In order to establish which spots were most responsible for this grouping into clusters, a statistical analysis (Mann-Whitney test, with a significance level (P) equal to or less than 0.05, by using the GraphPad software version Instat 3.00) and of fold-change criterion (> 1.5) were applied. With this double approach, two panels of spots were identified: a first panel consisting in 3 spots (panel 1) and a second panel consisting in 3 spots (panel 2) (Table 3).

Table 3

| Spot ID | Panel | Cluster A (%Vol) | Cluster B (%Vol) | P Value (<0.05) | Fold-change (>1.5) |
|---------|-------|------------------|------------------|-----------------|--------------------|
| 97 | 1 and 2 | 0.125 ± 0.035 | 0.210 ± 0.057 | <0.01 | ↑ 1.7 |
| 98 | 1 | 0.033 ± 0.003 | 0.046 ± 0.011 | <0.05 | ≅ |
| 288 | 2 | 0.224 ± 0.150 | 0.139 ± 0.099 | NS | ↓ 1.6 |
| 289 | 1 and 2 | 0.121 ± 0.025 | 0.214 ± 0.082 | <0.05 | ↑ 1.7 |

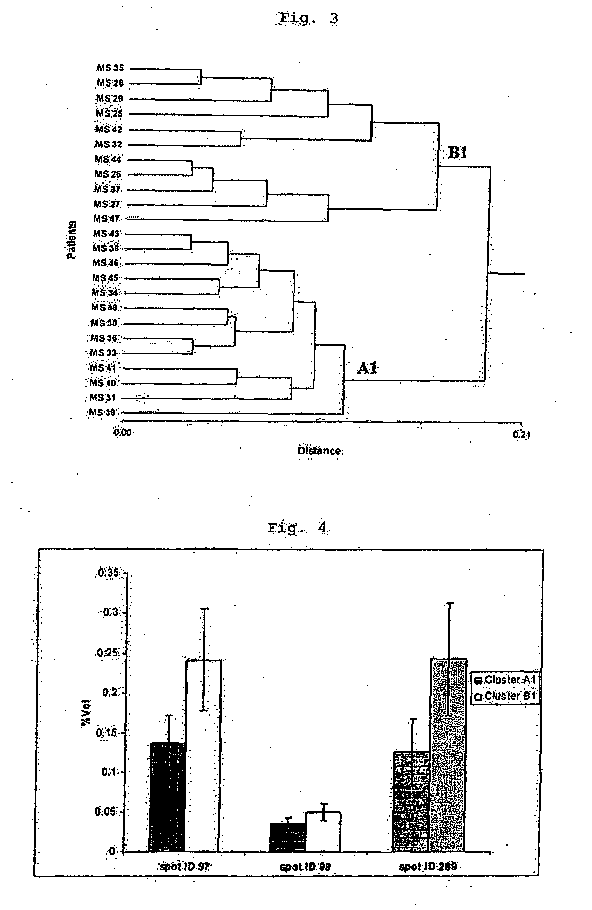

Subsequently, a further clusterization was performed by selecting the values of the Vol% of the spots of the panel 1. Following this analysis, a dendrogram is obtained, where the patients were grouped into two different clusters, renamed cluster A1 and cluster B1 (Fig. 3). These two new clusters are very similar to those obtained from the previous study (Fig. 2), except for the patients MS 34, MS 38, MS 40, and MS 43 who were moved from cluster B to cluster A (renamed Cluster B1 and Cluster A1, respectively). As regards outliers, MS 41, MS 31, and MS 45 are grouped in the cluster A1, while outliers MS 25 and MS 27 are found in cluster B1. The average values of the Vol% that the spot ID 98, 97, and 289 take in these 2 new clusters are set forth in Fig. 4.

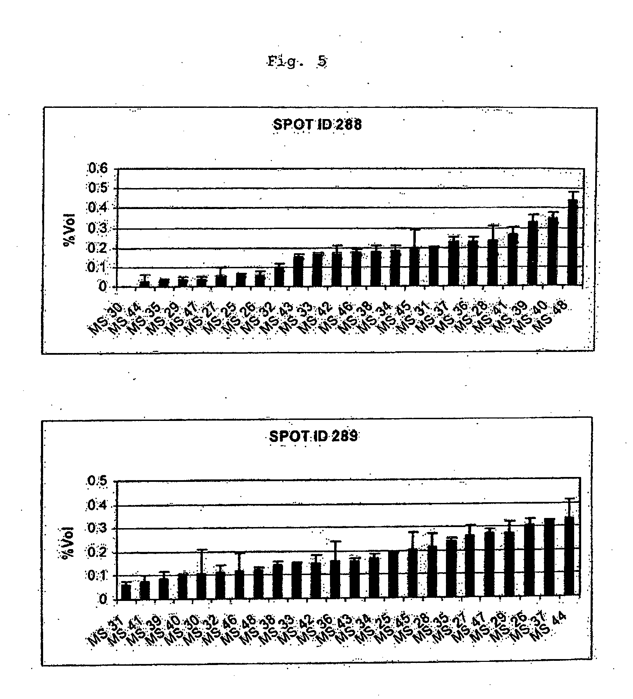

By performing the cluster analysis only on the spots ID 288 and 289, the detailed results of which for each patient are set forth in Fig. 5, a dendrogram was obtained, in which two main clusters are represented, referred to as Cluster G and Cluster Z and an outlier, patient MS 48 (Fig. 6). Furthermore, again with reference to the outlined spots ID 288 and 289, a post-test discriminant analysis was performed, to test the ability thereof to classify each subject in the pertinent population based on the volumes % measured (used software: StatistiXL). The overall ability of a correct prediction reaches 100% (Fig. 7).

**Example 4: Biomarkers-disease relationship**

All the patients included in the cluster B1 in the Example 3, except for the female patients MS 29, MS42 and MS 25, have a delta EDSS > 0, and after a two-year follow-up are subjected to therapy. EDSS variation was obtained by comparing the EDSS score upon lumber puncture and after two-year follow-up. All the patients included in the cluster A1 after two years are not so far undergoing any treatments, and the EDSS value remained unaltered post-collection.

Therefore, the patients included in the group B1 show a more aggressive disease course; on the contrary, patients included in the cluster A1 show a more benign disease course.

**Example 5: Mass spectrometry identification of the identified biomarkers**

The spot identified in Example 3 were manually cut from the corresponding gels and discolored overnight with a solution containing 40% ethanol in ammonium bicarbonate (25 mM); then they were washed twice with ammonium bicarbonate (25 mM), thrice with acetonitrile, then dried. Each gel fragment was rehydrated in ammonium bicarbonate (25 mM) containing 0.6 $\mu$g modified porcine trypsin, and a protease digestion was carried out overnight at 37° C. The resulting peptides were extracted by sonication in ammonium bicarbonate (25 mM), loaded on a ZORBAX 300 SB C18 RP column (75 pm x 150 mm, 3.5 $\mu$m particles, Agilent, Milan, Italy), and eluted with a 5%-80% acetonitrile gradient (containing 0.1% formic acid) at a 0.3 $\mu$l/min flow rate, by using a HP 1100 nanoLC system coupled to a XCT-Plus nanospray-ion trap mass spectrometer (Agilent)(LC-ESI MS/MS). The parameters used for the mass spectrometer were as follows: scan width = 100-2,200 m/z; scan rate = 8,100 m/z per s; gas flow rate = 5 l/min; temperature = 300° C;

capillary = 1.8 kV; skimmer = 40 V; ion charge control target (ICC) = 125,000; maximum build-up time = 300 ms. The positively charged peptides were automatically isolated and fragmented, and spectra were deconvoluted by using the DataAnalysis software (Bruker Daltonics, Bremen, Germany). The LC-ESI MS/MS mass data were used for searching the non-redundant NCBI protein sequence database through the Mascot research algorithm (http://www.matrixscience.com - mass tolerance of the monoisotopic peak: 1.8 Da for the parental ion, or 0.8 Da for the fragments; maximum number of uncut sites per peptide of 3). Carbamidomethylation of cysteines and oxidation of methionines were considered as permitted changes. Results with a Mowse score exceeding 47 were considered as significant (p < 0.05) (Mila et al., 2009).

The protein identity of the thus-obtained biomarkers is set forth in the following Table 4 and indicated by the corresponding sequence identifiers (SEQ ID NO).

Table 4

| SEQ. ID | Spot ID | LC-ESI MS/MS | *Accession number* | Number of Peptides | Coverage sequence | Apparent MW(KDa)/ pI | Mowse |
|---------|---------|--------------|--------------------|--------------------|--------------------|----------------------|-------|
| 1 | 97 | Gelsolin Isoform Precursor | gi4504165 | 21 | 33-748 | 85.6/5.9 | 415 |
| 2 | 98 | Gelsolin Isoform Precursor | gi4504165 | 6 | 148-738 | 85.6/5.9 | 117 |
| 3 | 288 | Vitamin D-binding protein | gi181482 | 13 | 52-440 | 53.0/5.4 | 291 |
| 4 | 289 | Vitamin D-binding protein | gi181482 | 20 | 52-471 | 52.9/5.4 | 480 |

Mass spectrometry analysis allowed identifying the spots ID 97 and 98 as gelsolin (SEQ. ID 1 and 2), and both spots ID 288 and 289 turn out to be attributable to DBP (SEQ. ID 3 and 4).

The following Tables set forth the protein sequence for the proteins identified in the spots ID 97, 98, 288, and 289.

```
Spot 97 SEQ. ID 1

  1    MAPHRPAPAL   LCALSLALCA    LSLPVRAATA   SRGASQAGAP   QGRVPEARPN
 51    SMVVEHPEFL   KAGKEPGLQI    WRVEKFDLVP   VPTNLYGDFF   TGDAYVILKT
101    VQLRNGNLQY   DLHYWLGNEC    SQDESGAAAI   FTVQLDDYLN   GRAVQHREVQ
151    GFESATFLGY   FKSGLKYKKG    GVASGFKHVV   PNEVVVQRLF   QVKGRRVVRA
201    TEVPVSWESF   NNGDCFILDL    GNNIHQWCGS   NSNRYERLKA   TQVSKGIRDN
251    ERSGRARVHV   SEEGTEPEAM    LQVLGPKPAL   PAGTEDTAKE   DAANRKLAKL
301    YKVSNGAGTM   SVSLVADENP    FAQGALKSED   CFILDHGKDG   KIFVWKGKQA
351    NTEERKAALK   TASDFITKMD    YPKQTQVSVL   PEGGETPLFK   QFFKNWRDPD
401    QTDGLGLSYL   SSHIANVERV    PFDAATLHTS   TAMAAQHGMD   DDGTGQKQIW
451    RIEGSNKVPV   DPATYGQFYG    GDSYIILYNY   RHGGRQGQII   YNWQGAQSTQ
501    DEVAASAILT   AQLDEELGGT    PVQSRVVQGK   EPAHLMSLFG   GKPMIIYKGG
551    TSREGGQTAP   ASTRLFQVRA    NSAGATRAVE   VLPKAGALNS   NDAFVLKTPS
601    AAYLWVGTGA   SEAEKTGAQE    LLRVLRAQPV   QVAEGSEPDG   FWEALGGKAA
651    YRTSPRLKDK   KMDAHPPRLF    ACSNKIGRFV   IEEVPGELMQ   EDLATDDVML
701    LDTWDQVFVW   VGKDSQEEEK    TEALTSAKRY   IETDPANRDR   RTPITVVKQG
751    FEPPSFVGWF   LGWDDDYWSV    DPLDRAMAEL   AA
```

Spot 98 SEQ ID. 2

| 1 | MAPHRPAPAL | LCALSLALCA | LSLPVRAATA | SRGASQAGAP | QGRVPEARPN |
|---|---|---|---|---|---|
| 51 | SMVVEHPEFL | KAGKEPGLQI | WRVEKFDLVP | VPTNLYGDFF | TGDAYVILKT |
| 101 | VQLRNGNLQY | DLHYWLGNEC | SQDESGAAAI | FTVQLDDYLN | GRAVQHREVQ |
| 151 | GFESATFLGY | FKSGLKYKKG | GVASGFKHVV | PNEVVVQRLF | QVKGRRVVRA |
| 201 | TEVPVSWESF | NNGDCFILDL | GNNIHQWCGS | NSNRYERLKA | TQVSKGIRDN |
| 251 | ERSGRARVHV | SEEGTEPEAM | LQVLGPKPAL | PAGTEDTAKE | DAANRKLAKL |
| 301 | YKVSNGAGTM | SVSLVADENP | FAQGALKSED | CFILDHGKDG | KIFVWKGKQA |
| 351 | NTEERKAALK | TASDFITKMD | YPKQTQVSVL | PEGGETPLFK | QFFKNWRDPD |
| 401 | QTDGLGLSYL | SSHIANVERV | PFDAATLHTS | TAMAAQHGMD | DDGTGQKQIW |
| 451 | RIEGSNKVPV | DPATYGQFYG | GDSYIILYNY | RHGGRQGQII | YNWQGAQSTQ |
| 501 | DEVAASAILT | AQLDEELGGT | PVQSRVVQGK | EPAHLMSLFG | GKPMIIYKGG |
| 551 | TSREGGQTAP | ASTRLFQVRA | NSAGATRAVE | VLPKAGALNS | NDAFVLKTPS |
| 601 | AAYLWVGTGA | SEAEKTGAQE | LLRVLRAQPV | QVAEGSEPDG | FWEALGGKAA |
| 651 | YRTSPRLKDK | KMDAHPPRLF | ACSNKIGRFV | IEEVPGELMQ | EDLATDDVML |
| 701 | LDTWDQVFVW | VGKDSQEEEK | TEALTSAKRY | IETDPANRDR | RTPITVVKQG |

| 751 | FEPPSFVGWF | LGWDDDYWSV | DPLDRAMAEL | AA |
|---|---|---|---|---|

Spot 288 SEQ. ID 3

| 1 | MKRVLVLLLA | VAFGHALERG | RDYEKNKVCK | EFSHLGKEDF | TSLSLVLYSR |
|---|---|---|---|---|---|
| 51 | KFPSGTFEQV | SQLVKEVVSL | TEACCAEGAD | PDCYDTRTSA | LSAKSCESNS |
| 101 | PFPVHPGTAE | CCTKEGLERK | LCMAALKHQP | QEFPTYVEPT | NDEICEAFRK |
| 151 | DPKEYANQFM | WEYSTNYEQA | PLSLLVSYTK | SYLSMVGSCC | TSASPTVCFL |
| 201 | KERLQLKHLS | LLTTLSNRVC | SQYAAYGEKK | SRLSNLIKLA | QKVPTADLED |
| 251 | VLPLAEDITN | ILSKCCESAS | EDCMAKELPE | HTVKLCDNLS | TKNSKFEDCC |
| 301 | QEKTAMDVFV | CTYFMPAAQL | PELPDVRLPT | NKDVCDPGNT | KVMDKYTFEL |
| 351 | SRRTHLPEVF | LSKVLEPTLK | SLGECCDVED | STTCFNAKGP | LLKKELSSFI |
| 401 | DKGQELCADY | SENTFTEYKK | KLAERLKAKL | PEATPTELAK | LVNKRSDFAS |
| 451 | NCCSINSPPL | YCDSEIDAEL | KNIL | | |

Spot 289 SEQ. ID 4

| 1 | MKRVLVLLLA | VAFGHALERG | RDYEKNKVCK | EFSHLGKEDF | TSLSLVLYSR |
|---|---|---|---|---|---|
| 51 | KFPSGTFEQV | SQLVKEVVSL | TEACCAEGAD | PDCYDTRTSA | LSAKSCESNS |
| 101 | PFPVHPGTAE | CCTKEGLERK | LCMAALKHQP | QEFPTYVEPT | NDEICEAFRK |
| 151 | DPKEYANQFM | WEYSTNYEQA | PLSLLVSYTK | SYLSMVGSCC | TSASPTVCFL |
| 201 | KERLQLKHLS | LLTTLSNRVC | SQYAAYGEKK | SRLSNLIKLA | QKVPTADLED |
| 251 | VLPLAEDITN | ILSKCCESAS | EDCMAKELPE | HTVKLCDNLS | TKNSKFEDCC |
| 301 | QEKTAMDVFV | CTYFMPAAQL | PELPDVRLPT | NKDVCDPGNT | KVMDKYTFEL |
| 351 | SRRTHLPEVF | LSKVLEPTLK | SLGECCDVED | STTCFNAKGP | LLKKELSSFI |
| 401 | DKGQELCADY | SENTFTEYKK | KLAERLKAKL | PEATPTELAK | LVNKRSDFAS |
| 451 | NCCSINSPPL | YCDSEIDAEL | KNIL | | |

**Example 6: calculation of the Vol% ratio of the spots ID 288 and 289**

The combination of the Vol% values of the spots ID 289 and 288 can be expressed by two linear functions:

$$f1 = 33.813 * (Vol\% \ spot \ ID \ 288) + 48.942 * (Vol\% \ spot \ ID \ 289) - 7.155;$$

$$f2 = 103.508 * (Vol\% \ spot \ ID \ 288) + 42.871 * (Vol\% \ spot \ ID \ 289) - 15.248;$$

and represents a tool to correctly cluster (100%) the entire population into two main groups (groups G and Z) and to identify the subject MS 48 as an outlier (Fig. 7). The straight line separating the two clusters is defined by the function $y = 1.5629x - 1.7155$.

the identification of the spots ID 288 and 289 by mass spectrometry and the difference of the % volume values were validated by a 2-DE western blotting technique on a pool of samples.

In order to perform the 2DE Western blotting experiments, the cerebral fluid proteins were denatured, then separated on 2DE gel, as described above.

After the run, the gels were immediately immersed in an aqueous solution consisting in Tris 25 mM, 40 mM 6-aminohexanoic acid, and 20% v/v methanol, ensuring a final pH value of 9.4. The thus-separated proteins were transferred on nitrocellulose membranes (Hybond C-extra, with pores of 0.45 micrometers; GE Healthcare, Uppsala, Sweden) applying a "semi-dry" type transfer. After transferring, the membranes were incubated for at least 15 hours at 4° C in a blocking solution composed of TBS (in the presence of 0.1% w/v Tween 20 (T-TBS) and 5% w/v of bovine serum albumin. TBS-T was further used in the washing steps, to remove possible non-specific bonds with the antibody. As the main recognition antibody, a goat polyclonal anti-DBP antibody (SIGMA ALDRICH) was used at a 1:500 dilution in 0.1% TBST.

As the secondary detection antibody, a HRP-conjugated (horseradish peroxidase) anti-goat was used at a 1:10.000 dilution in 0.1% TBST; the membrane was then incubated with a specific chemoluminescent substrate provided by the ECL Western Blotting kit (Pierce, Euroclone). Images relating to the proteins displayed following exposure on film were taken through the ImageMaster Labscan V3.0 (GE Healthcare, Uppsala, Sweden).

Subsequently, phosphoproteomic assays were conducted, to assess the possible involvement of the phosphorylation state in the different expression of the spots among the patients under examination. The gels were stained with ProQ Diamond (Molecular Probes Inc, Eugene, OR) [Agrawal and Thelen, 2005) before being stained with Sypro Ruby, but no phosphorylation was shown in the identified spots.

By the 2-DE technique, allowing a separation of the proteins based on their isoelectric point, it has been shown that the differential migration of the spots ID 288 and 289 in the horizontal direction was due to their different glycosylation degree. In particular, the spot migrated in a more acidic position (ID 289) would contain the glycosylated isoform.

Bibliography

Agrawal G.K., Thelen J.J. "Development of a simplified, economical polyacrylamide gel staining protocol for phosphoproteins" Proteomics 5: 4684-4688 (2005).

Bowser R., Cudkowicz M., Kaddurah-Daouk R. "Biomarkers for amyotrophic lateral sclerosis" Expert Rev. Mol. Diagn. 6: 387 (2006).

Disanto G., Ramagopalan S.V., Para A.AND., Handunnetthi L. "The emerging role of vitamin D binding protein in multiple sclerosis" J. Neurol. 258(3): 353-8 (2011).

Freedman M.S. "Long-term follow-up of clinical trials of multiple sclerosis therapies" Neurology 76 (1 Suppl 1): S26-34 (2011).

Ghezzi A. Cazzullo C.L. et al. "La Sclerosi Multipla ad onset precoce" Atti Congresso SINPI, S. Marino (1980).

Kulakowska A., Drozdowski W., Sadzynski A., Bucki R., Janmey P.A. "Gelsolin concentration in cerebrospinal fluid from patients with multiple sclerosis and other neurological disorders" European J. Neurology 15: 584-588 (2008).

Kulakowska A., Ciccarelli N.J., Wen Q., Mroczko B., Drozdowski Szmitkowski M., Janmey P.A., Bucki R. "Hypogelsolinemia, a disorder of the extracellular actin scavenger system, in patients with multiple sclerosis" BMC Neurology 10: 107 (2010).

Lacomblez L., Bensimon G., Leigh P.N., Guillet P., Meininger V. "Dose-ranging study of riluzole in amyotrophic lateral sclerosis" Amyotrophic Lateral Sclerosis/Riluzole Study Group II. Lancet, 347: 1425 (1996).

Lublin F.D., Reingold S.C. "Defining the clinical course of multiple sclerosis: results of an international survey". National Multiple Sclerosis Society (USA) Advisory Committee on Clinical Trials of New Agents in Multiple Sclerosis. Neurology 46(4): 907-11 (1996).

Poser C., et al. "The course of multiple sclerosis" Archives of Neurology 42: 10-35 (1985).

McDonald WI, Compston A, Edan G, Goodkin D, Hartung HP, Lublin FD, McFarland HF, Paty DW, Polman CH, Reingold SC, Sandberg-Wollheim M, Sibley W, Thompson A, van den Noort S, Weinshenker BY, Wolinsky JS. "Recommended diagnostic criteria for multiple sclerosis: guidelines from the International Panel on the diagnosis of multiple sclerosis" Ann. Neurol. 50: 121-127 (2001).

Rithidech K.N., Honikel L., Milazzo M., Madigan D., Troxell R., Krupp L.B. "Protein expression profiles in pediatric multiple sclerosis: potential biomarkers" Mult. Scler. 15(4):455-64 (2009).

Robotti A., Natale M., Giuliano Albo A., Lis K., Perga S., Marnetto F., Gilli F., Bertolotto A. "Acute-phase proteins investigation based on lectins affinity capture prior to 2-DE separation: Application to serum from multiple sclerosis patients" Electrophoresis 17: 2882-2893 (2010).

Shaw P.J. and Williams R. "Serum and cerebrospinal fluid biochemical markers of ALS". Amyotroph Lateral Scler Other Motor Neuron Disord. 1: S61 (2000).

## Claims

1. A method of diagnosis and assessment of the progression status of Multiple Sclerosis, comprising:

   i. measuring the levels of Vitamin D Binding Protein ID 289, glycosylated isoform, SEQ. ID 4 and Vitamin D Binding Protein ID 288, SEQ. ID 3 in a sample C, where, ID 289 and ID 288 represent modified *Vitamin D binding protein* (DBP) isoforms, where said isoform ID 289 is a glycosylated isoform of DBP;
   ii. obtaining the coordinates f1 and f2, where f1= 33.813 * ID 288 level + 48.942 * ID 289 level - 7.155 and f2= 103.508 * ID 288 level + 42.871 * ID 289 level - 15.248;
   iii. positioning in the Cartesian plane the point C(f1, f2) and assessing the position thereof relative to the straight line y = 1.5629x - 1;
   iv. where C(f1, f2)> y = 1.5629x - 1, the sample C will be assigned to the MSSP (secondary progressive MS) or MSPP (primary progressive MS) group; where C(f1, f2)< y = 1.5629x - 1, the sample C will be assigned to the MSRR group (relapsing - remitting MS).

2. The method in accordance with the claim 1, further comprising:

   i. measuring the ID 97, SEQ. ID 1 and/or ID 98, SEQ. ID 2 level in a sample C and in a control MSRR, where ID97 and ID98 are two gelsolin isoforms separated by SDS-PAGE in a 12.5% gel;

ii.obtaining the value G = ID 97 level + ID 98 level, or G = ID 97 level or G = ID 98 level in the sample ($G_C$) and in the control ($G_{CtrlMSRR}$) ;
iii. where C(f1, f2)> y = 1.5629x - 1 and Gc > $G_{CtrlMSRR}$ in a statistically significant manner, assigning the sample C to the MSSP MSPP group; where C(f1, f2)< y = 1.5629x - 1 and $G_C$ = - $G_{CtrlMSRR}$, assigning the sample C to the MSRR group.

**3.** The method in accordance with one of the claims 1 or 2, further comprising the assessment of further biomarkers selected in the group comprising: complement factor C3, complement factor C4, beta-2-microglobulin, Clusterin, Prostaglandin H2 D isomerase, Haptoglobin, Immunoglobulins, Apolipoprotein E, Beta fibrinogen, alpha 2 macroglobulin, Fetuin A, serin protease inhibitor, Tansthyretin, Albumin, Transferrin, Apolipoprotein D, Retinol binding protein, Apolipoprotein A4, SPARC-like protein, Autotaxin t, Pigment epithelium derived factor, Angiotensinogen, Chromogranin A, Tuberous sclerosis complex 2, Cystatin C, Ceruloplasmin, Superoxide dismutase 1, Actin, Beta V Spectrin, Cartilage acidic protein 1, Fibulin 1, Calsyntenin 3, Contactin 1, Xantin dehydrogenase/oxidase, RNA binding motif protein 7, Ribonuclease 1, neuroendocrine protein 7 B2, alpha 1 Antitrypsin, alpha 1 Antichymotrypsin, Kallikrein 6, EGF-containing fibulin-like extracellular matrix protein 1, Plasminogen, Antithrombin III, Dickkofp relata protein 3, neuronal pentraxin receptor, Tetranectin.

**4.** The method according to one of the claims 1 to 3, where said proteins are detected and quantified by techniques for protein analysis, preferably selected from the group comprising Western-Blot, 2DE gel electrophoresis, ELISA (Enzyme-Linked Immunosorbent assay), RIA (Radioimmunoassay), Competitive EIA (Competitive Enzyme Immunoassay), DAS-ELISA (Double Antibody Sandwich-ELISA), other immunocytochemical or immunohistochemical techniques optionally combined with HPLC or mass spectrometry.

**Patentansprüche**

**1.** Verfahren zur Diagnose und Beurteilung des Fortschrittsstatus von Multipler Sklerose, umfassend:

i. Messen der Spiegel von Vitamin-D-bindendem Protein ID 289, glykosylierte Isoform, SEQ. ID 4, und Vitamin-D-bindendem Protein ID 288, SEQ. ID 3, in einer Probe C, wobei ID 289 und ID 288 modifizierte Vitamin-D-bindende Protein (DBP)-Isoformen darstellen, wobei die Isoform ID 289 eine glykosylierte Isoform von DBP ist;
ii. Erhalten der Koordinaten f1 und f2, wobei f1= 33,813 * ID 288 Spiegel + 48,942 * ID 289 Spiegel - 7,155 und f2= 103,508 * ID 288 Spiegel + 42,871 * ID 289 Spiegel - 15,248;
iii. Positionieren des Punkts C (f1, f2) in dem kartesischen Koordinatensystem und Beurteilen der Position davon in Bezug auf die gerade Linie y = 1,5629x - 1;
iv. Zuweisen der Probe C zu der MSSP (sekundäre progrediente MS)-Gruppe oder MSPP (primäre progrediente MS)-Gruppe, wo C (f1, f2)> y = 1,5629x - 1; Zuweisen der Probe C zu der MSRR (rezidivierende - remittierende MS)-Gruppe, wo C(f1, f2)< y = 1,5629x - 1.

**2.** Verfahren nach Anspruch 1, weiter umfassend:

i. Messen der ID 97, SEQ. ID 1, und/oder ID 98, SEQ. ID 2, Spiegel in einer Probe C und in einer Kontroll-MSRR, wobei ID97 und ID98 zwei Gelsolin-Isoformen sind, die durch SDS-PAGE in einem 12,5% Gel getrennt werden;
ii. Erhalten des Werts G = ID 97 Spiegel + ID 98 Spiegel, oder G = ID 97 Spiegel oder G = ID 98 Spiegel in der Probe ($G_C$) und in der Kontrolle ($G_{CtrlMSRR}$);
iii. Zuweisen der Probe C zu der MSSP MSPP Gruppe, wo C (f1, f2)> y = 1,5629x - 1 und $G_C$ > $G_{CtrlMSRR}$ in einer statistisch signifikanten Weise;
Zuweisen der Probe C der MSRR Gruppe, wo C(f1, f2)< y = 1,5629x - 1 und $G_C$ = $G_{CTrlMSRR}$.

**3.** Verfahren nach Anspruch 1 oder 2, weiter umfassend die Beurteilung weiterer Biomarker ausgewählt aus der Gruppe umfassend:

Komplementfaktor C3, Komplementfaktor C4, beta-2-Mikroglobulin, Clusterin, Prostaglandin H2 D-Isomerase, Haptoglobin, Immunglobuline, Apolipoprotein E, beta-Fibrinogen, alpha-2-Makroglobulin, Fetuin A, Serin-Protease-Inhibitor, Transthyretin, Albumin, Transferrin, Apolipoprotein D, Retinol-bindendes Protein Apolipoprotein A4, SPARC-artiges Protein, Autotaxin t, pigmentepithelabgeleiteter Faktor, Angiotensinogen, Chromogranin A, tuberöser Sklerosekomplex 2, Cystatin C, Ceruloplasmin, Superoxiddismutase 1, Actin, Beta V Spectrin, Cartilage-acidic-Protein 1, Fibulin 1, Calsyntenin 3, Contactin 1, Xantin Dehydrogenase / Oxidase, RNA bindendes Motiv Protein 7, Ribonuclease 1, neuroendokrines Protein 7 B2, alpha 1 Antitrypsin, alpha 1 Antichymotrypsin, Kallikrein 6 EGFhaltiges fibulinartiges extrazelluläres Matrixprotein 1, Plasminogen, Antithrombin III, Dickkopf-ähnliches Protein 3, neuronaler Pentraxin-Rezeptor, Tetranectin.

**4.** Verfahren nach einem der Ansprüche 1 bis 3, wobei die Proteine detektiert und quantifiziert werden durch Techniken zur Proteinanalyse, bevorzugt ausgewählt aus der Gruppe bestehend aus Western-Blot, 2D-Gelelektrophorese, ELISA (Enzyme Linked Immunosorbent Assay), RIA (Radioimmunassay), kompetitiver EIA (kompetitiver Enzymimmunassay), DAS-ELISA (Doppelantikörper-Sandwich-ELISA), andere immunzytochemische oder immunhistochemische Techniken, gegebenenfalls kombiniert mit HPLC oder Massenspektrometrie.

## Revendications

**1.** Méthode de diagnostic et de détermination de l'état de progression de la sclérose en plaques, comprenant :

    i. la mesure des niveaux de l'isoforme glycosylée de la protéine ID 289 se liant à la vitamine D, SEQ ID NO : 4, et de la protéine ID 288 se liant à la vitamine D, SEQ ID NO : 3, dans un échantillon C, où ID 289 et ID 288 représentent des isoformes modifiées de protéine se liant à la vitamine D (DBP), ladite isoforme ID 289 étant une isoforme glycosylée de la DBP ;
    ii. l'obtention de coordonnées f1 et f2, où f1 = 33,813 x niveau d'ID 288 + 48,942 x niveau d'ID 289 - 7,155 et f2 = 103,508 x niveau d'ID 288 + 42,871 x niveau d'ID 289 - 15,248 ;
    iii. le positionnement, dans le plan cartésien, du point C(f1, f2), et la détermination de sa position par rapport à la droite y = 1,5629x - 1 ;
    iv. lorsque C(f1, f2) > y = 1,5629x - 1, l'échantillon C sera attribué à une MSSP (MS progressive secondaire) ou une MSPP (MS progressive primaire) ; lorsque C(f1, f2) < y = 1,5629x - 1, l'échantillon C sera attribué au groupe MSRR (MS récurrente rémittente).

**2.** Méthode selon la revendication 1, comprenant en outre :

    i. la mesure du niveau d'ID 97, SEQ ID NO : 1, et/ou d'ID 98, SEQ ID NO : 2, dans un échantillon C et dans une MSRR témoin, où ID97 et ID98 sont deux isoformes de gelsoline séparées par SDS-PAGE dans un gel à 12,5 % ;
    ii. l'obtention de la valeur G = niveau d'ID 97 + niveau d'ID 98, ou G = niveau d'ID 97 ou G = niveau d'ID 98 dans l'échantillon ($G_C$) et dans le témoin ($G_{CtrlMSRR}$) ;
    iii. lorsque C(f1, f2) > y = 1,5629x - 1 et $G_C$ > $G_{CtrlMSRR}$ d'une manière statistiquement significative, l'attribution de l'échantillon C au groupe MSSP MSPP ; lorsque C(f1, f2) < y = 1,5629x - 1 et $G_C$ = $G_{CtrlMSRR}$, l'attribution de l'échantillon C au groupe MSRR.

**3.** Méthode selon l'une des revendications 1 et 2, comprenant en outre la détermination d'autres marqueurs biologiques choisis dans le groupe comprenant : le facteur de complément C3, le facteur de complément C4, la bêta-2-microglobuline, la clustérine, la prostaglandine H2 D isomérase, l'haptoglobine, les immunoglobulines, l'apolipoprotéine E, le fibrinogène bêta, la macroglobuline alpha 2, la féreine A, l'inhibiteur de sérine protéase, la tansthyrétine, l'albumine, la transferrine, l'apolipoprotéine D, la protéine se liant au rétinol, l'apolipoprotéine A4, la protéine de type SPARC, l'autotaxine t, le facteur dérivé d'épithélium pigmentaire, l'angiotensinogène, la chomogranine A, le complexe de sclérose tubéreuse 2, la cystatine C, la céruloplasmine, la superoxyde dismutase 1, l'actine, la spectrine bêta V, la protéine acide de cartilage 1, la fibuline 1, la calsynténine 3, la contactine 1, la xanthine déshydrogénase/oxydase, la protéine de motif se liant à l'ARN 7, la ribonucléase 1, la protéine neuroendocrinienne 7 B2, l'antitrypsine alpha 1, l'antichymotrypsine alpha 1, la kallikréine 6, la protéine de matrice extracellulaire analogue à la fibuline contenant de l'EGF 1, le plasminogène, l'antithrombine III, la protéine de type Dickkopf 3, le récepteur de pentraxine neuronale, la tétranectine.

**4.** Méthode selon l'une des revendications 1 à 3, dans laquelle lesdites protéines sont détectées et quantifiées par des techniques pour l'analyse de protéines, de préférence choisies dans le groupe comprenant le transfert Western, l'électrophorèse sur gel 2DE, l'ELISA (immunodosage enzymatique), le RIA (radioimmunodosage), l'EIA compétitif (immunodosage enzymatique compétitif), le DAS-ELISA (ELISA en sandwich à anticorps double), d'autres techniques immunocytochimiques ou immunohistochimiques éventuellement combinées à une HPLC ou une spectrométrie de masse.

Fig. 1

Fig. 2

Fig. 3

Fig. 4

Fig. 5

Fig. 6

Fig. 7

Fig. 8

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- SE 2006000866 W
- WO 02059604 A

**Non-patent literature cited in the description**

- **OTTERVALD J et al.** *Journal of Proteomics,* 2010, vol. 73, 1117-1132
- **DISANTO et al.** *Journal of Neurology,* 2010, vol. 258, 353-358
- **KARLIN ; ALTSCHUL.** *Proc. Natl. Acad. Sci. USA,* 1990, vol. 87, 2264-2268
- **KARLIN ; ALTSCHUL.** *Proc. Natl. Acad. Sci. USA,* 1993, vol. 90, 5873-5877
- **ALTSCHUL et al.** *J. Mol. Biol.,* 1990, vol. 215, 403-410
- **DEVEREUX et al.** *Nucleic Acids Research,* 1984, vol. 12, 387
- **BOWIE et al.** *Science,* 1990, vol. 247, 1306-1310
- **AGRAWAL G.K. ; THELEN J.J.** Development of a simplified, economical polyacrylamide gel staining protocol for phosphoproteins. *Proteomics,* 2005, vol. 5, 4684-4688
- **BOWSER R. ; CUDKOWICZ M. ; KADDU-RAH-DAOUK R.** Biomarkers for amyotrophic lateral sclerosis. *Expert Rev. Mol. Diagn.,* 2006, vol. 6, 387
- **DISANTO G. ; RAMAGOPALAN S.V. ; PARA A.AND. ; HANDUNNETTHI L.** The emerging role of vitamin D binding protein in multiple sclerosis. *J. Neurol.,* 2011, vol. 258 (3), 353-8
- **FREEDMAN M.S.** Long-term follow-up of clinical trials of multiple sclerosis therapies. *Neurology,* 2011, vol. 76 (1), 26-34
- **GHEZZI A. ; CAZZULLO C.L. et al.** La Sclerosi Multipla ad onset precoce. *Atti Congresso SINPI, S. Marino,* 1980
- **KULAKOWSKA A. ; DROZDOWSKI W. ; SADZYN-SKI A. ; BUCKI R. ; JANMEY P.A.** Gelsolin concentration in cerebrospinal fluid from patients with multiple sclerosis and other neurological disorders. *European J. Neurology,* 2008, vol. 15, 584-588
- **KULAKOWSKA A. ; CICCARELLI N.J. ; WEN Q. ; MROCZKO B. ; DROZDOWSKI SZMITKOWSKI M. ; JANMEY P.A. ; BUCKI R.** Hypogelsolinemia, a disorder of the extracellular actin scavenger system, in patients with multiple sclerosis. *BMC Neurology,* 2010, vol. 10, 107
- **LACOMBLEZ L. ; BENSIMON G. ; LEIGH P.N. ; GUILLET P. ; MEININGER V.** Dose-ranging study of riluzole in amyotrophic lateral sclerosis. *Amyotrophic Lateral Sclerosis/Riluzole Study Group II. Lancet,* 1996, vol. 347, 1425
- **LUBLIN F.D. ; REINGOLD S.C.** Defining the clinical course of multiple sclerosis: results of an international survey. *Neurology,* 1996, vol. 46 (4), 907-11
- **POSER C. et al.** The course of multiple sclerosis. *Archives of Neurology,* 1985, vol. 42, 10-35
- **MCDONALD WI ; COMPSTON A ; EDAN G ; GOODKIN D ; HARTUNG HP ; LUBLIN FD ; MC-FARLAND HF ; PATY DW ; POLMAN CH ; REIN-GOLD SC.** Recommended diagnostic criteria for multiple sclerosis: guidelines from the International Panel on the diagnosis of multiple sclerosis. *Ann. Neurol.,* 2001, vol. 50, 121-127
- **RITHIDECH K.N. ; HONIKEL L. ; MILAZZO M. ; MA-DIGAN D. ; TROXELL R. ; KRUPP L.B.** Protein expression profiles in pediatric multiple sclerosis: potential biomarkers. *Mult. Scler.,* 2009, vol. 15 (4), 455-64
- **ROBOTTI A. ; NATALE M. ; GIULIANO ALBO A. ; LIS K. ; PERGA S. ; MARNETTO F. ; GILLI F. ; BERTOLOTTO A.** Acute-phase proteins investigation based on lectins affinity capture prior to 2-DE separation: Application to serum from multiple sclerosis patients. *Electrophoresis,* 2010, vol. 17, 2882-2893
- **SHAW P.J. ; WILLIAMS R.** Serum and cerebrospinal fluid biochemical markers of ALS. *Amyotroph Lateral Scler Other Motor Neuron Disord.,* 2000, vol. 1, S61